# EUROPEAN PATENT SPECIFICATION

(11) **EP 0 701 818 B1**
(45) Date of publication and mention of the grant of the patent: **30.07.2003**
(21) Application number: 95111577.3
(22) Date of filing: 04.05.1989
(51) Int. Cl.: A61K 39/395, C12N 9/00

(54) **Therapeutic methods using catalytic antibodies**
Heilungsverfahren unter Verwendung katalytischer Antikörper
Procédés thérapeutiques utilisant des anticorps catalytiques

(30) Priority: 04.05.1988 US 190271
(43) Date of publication of application: 20.03.1996
(62) Divisional of application: 89906520.5
(73) Proprietor: IGEN International, Inc., Gaithersburg, MD 20877 (US)
(72) Inventor: Powell, Michael J., Gaithersburg, Maryland 20852 (US); Massey, Richard J., Rockville, Maryland 20852 (US); Rees, Anthony R., Rockville, Maryland 20852 (US)
(74) Representative: Schlich, George William

(56) References cited:
- EP-A- 0 251 093
- EP-A- 0 260 939
- EP-A- 0 305 870
- WO-A-85/02414
- WO-A-90/13661
- BIOTECHNOLOGY, vol. 5, no. 8, NEW YORK US, page 767 XP002028355 VAN BRUNT J.: "Antibodies find a new role-As enzymes"
- RECHERCHE, LA, vol. 18, no. 187, PARIS FR, pages 516-517, XP002028356 LETHUILLIER A.: "Les abzymes: des anticorps convertis en enzymes"
- SCIENTIFIC AMERICAN, vol. 258, no. 3, NEW YORK US, pages 42-50, XP002028357 LERNER A.L. ET AL: "catalytic antibodies"

## Description

### FIELD OF THE INVENTION

This invention relates generally to methods for achieving changes in biological function by enhancing the rate of cleavage or formation of specific bonds in biomolecules in vivo. More particularly, this invention relates to enhancing the rate of cleavage or formation of specific amide, peptide, or ester bonds within proteins by introducing into an animal an antibody capable of enhancing the said rate of cleavage or formation, or by introducing into an animal an immunogen which elicits the desired rate-enhancing antibody.

Several publications are referenced in this application by Arabic numerals within parentheses. Full citation for these references are found at the end of the specification immediately preceding the claims. The references more fully describe the state of the art to which this invention pertains.

### BACKGROUND OF THE INVENTION

There are numerous enzymes which have been identified as capable of catalyzing various chemical reactions. Similarly, it has been discovered that antibodies can be elicited to catalyze a variety of chemical reactions (U.S. App. Ser. No. 674,253). It is well known that antibodies and enzymes share a fundamental similarity in that both are specialized proteins that bind to other molecules. However, there are important physiological differences between antibodies and enzymes.

Antibodies typically bind to a molecule or antigen so that the antigen is marked as foreign to the organism that produced the antibody. The binding of the antibody to the antigen enables the antigen to be removed from the organism. Enzymes are biological catalysts which bind a molecule in such a way that the activation energy of a reaction involving a molecule or substrate is lowered, thereby increasing the rate of the reaction.

Linus Pauling hypothesized that there are two types of interactions between proteins and the molecules that bind them. Antibodies bind molecules in their ground states most strongly while enzymes bind molecules in higher energy states most strongly.

Pauling attempted to explain the mechanism of enzyme catalysis based upon such binding. During the course of the chemical reaction, the reactants undergo one or more transitions through intermediate structures or transition states which are energically less favorable than either the reactant or the product. The hydrolysis reaction of a peptide linkage or an ester bond in an aqueous medium passes through a tetrahedral carbon transition state. In the transition state, a tetrahedral carbon atom is bonded to: a carbon atom of the acid portion of the peptide linkage or ester bond; two oxygen atoms, one corresponding to the carbonyl group and the other corresponding to a hydroxyl ion or water molecule of the medium; and either the oxygen atom of the alcohol portion of an ester or the nitrogen atom of the amine portion of the peptide linkage. The transition state can be neither isolated nor detected since it exists for only about 10⁻¹³ seconds.

In molecular terms, these transition states reflect changes in bond lengths and bond angles as well as the formation and breakage of bonds. The energy required to achieve a transition state is denoted as the activation energy which may also be considered as the difference in energy between the energy of the transition state and the energy of the reactants. According to Pauling's hypothesis, an enzyme preferentially binds the transition state of a reaction, thereby stabilizing it relative to the substrate and products and reducing the activation energy of the reaction, thus increasing the reaction rate.

By extending this explanation, Pauling also predicted that stable analogs of a transition state would bind tightly to an enzyme. In a discussion of substrate distortion as one of several possible sources of rate enhancement by enzymes, it has been suggested that the term "transition state analog" might be used to describe an inhibitor of this kind (1).

Pauling's prediction has become the basis for the now well established approach to enzyme inhibitor design. The strategy for designing enzyme inhibitors has suggested a strategy for preparing catalytic antibodies whereby antigens are designed based upon mechanistic principles so that antibodies raised in response to such antigens will catalyze a chemical reaction by carrying out the reaction mechanism implicit in the design of the antigen. This strategy has been attempted a number of times.

For example, a transition state analog mimicking the transition state for ester bond cleavage was used to elicit a monoclonal antibody which acted as a catalytic esterase (2). Specifically, the monoclonal antibody elicited accelerated by a factor of 15,000 the hydrolysis of an aryl ester of acetic acid.

In another example, a transition state analog mimicking an intramolecular 6-member ring cyclization transition state was used to elicit a monoclonal antibody which acted as a stereospecific, enzyme-like catalyst (3). Specifically, the monoclonal antibody so elicited accelerated, by about a factor of 800, the formation of a single enantiomer of a δ-lactone from the corresponding racemic δ-hydroxyester.

Similarly, monoaryl phosphonamidate esters, designated as analogs of the transition state in the hydrolysis of aryl amides, were synthesized and used as haptens to elicit specific monoclonal antibodies capable of catalyzing the hydrolysis of aryl amides (4). Certain of the antibodies elicited were reportedly found to be catalytic and selective for the hydrolysis of particular aryl esters, with a rate acceleration of 250,000.

Phosphonamidates or phosphonate analog-ligands having conformations that substantially correspond to the conformation of a hydrolytic transition state of an amide or ester ligand and which have been used to produce antibodies are described in U.S. Patent 4,659,567 to Tramontano et al. (Tramontano). Antibodies so produced purportedly include a paratope that binds to and stabilizes the tetrahedral carbon atom of the ester hydrolysis transition state of the ligand to hydrolyze the ligand at a predetermined site.

Analog-ligands which can be used to produce antibody catalysts for the hydrolysis of esters and amides are also described in European Patent Application 0,251,093 of Kollmorgen Corp (Kollmorgen).

Analog ligands have been designed in accordance with a rational design approach which maximizes stabilization of the transition state and optimizes atomic relationships within the proteolytic transition state analog, thereby enabling the elicitation of antibodies capable of producing the two dramatic effects of enzyme catalysis, i.e., molecular recognition and rate acceleration. Antigens, immunogens, and immunological processes for the production of catalytic antibodies are disclosed in commonly assigned parent application Ser. No. 190,271, filed May 4, 1988.

It is known that antibodies raised against peptides are able to bond to the same sequence when the latter are located within an intact protein. For example, antibodies elicited against a peptide comprising amino acids 1-15 of tumor necrosis factor (TNF) are able to bind to native tumor necrosis factor and in doing so, inhibit its interaction with a cell surface receptor (1). Similarly, antibodies against a peptide comprising amino acids of the gp 120 coat protein from HIV cross-react with the intact virus and inhibit the interaction of the virus with its cellular receptor, CD4 (2). In another example, monoclonal antibodies raised against a peptide comprising amino acids 67-83 of hen egg lysozyme were able to cross-react with the intact protein and are able to recognize other avian species of lysozyme whose sequences within the epitope are substantially similar (3).

While methods for preparing catalytic antibodies have been described, and while methods for binding noncatalytic antibodies to antigens or substrates of interest have been described, the art has heretofore not provided therapeutic methods for directing rate-enhancing antibodies against selected target biomolecules. Moreover, the art has not provided methods for decreasing or otherwise controlling the biological effects of certain undesirable proteins, e.g., tumor necrosis factor, nor has the art provided methods for reducing the symptoms of allergies, reducing the infectivity of HIV virus, decreasing the effects of hypertension and other disease and physiological conditions which can be controlled by cleaving or forming certain biomolecules.

Scientific American, 258, pages 42-50 (1988) suggests using catalytic antibodies to cleave viral coat proteins.

EP-A-0260939 proposes catalytic antibodies for cleavage of proteins, such as by cleavage of amide or ester bonds.

Other applications of catalytic antibodies, namely carbohydrate degradation, treatment of disease and anti-cancer therapy, are mentioned generally in EP-A-0251093.

The art has not however provided reliable methods for the activation of prodrugs using catalysts to release the active drug form from the prodrug.

### Objects of The Invention

It is a primary object of this invention to provide a pharmaceutical composition for enhancing the rate of cleavage or formation of a specific amide, peptide, ester, or glycosidic bond within biomolecules in vivo.

It is a further object of the invention to provide a pharmaceutical composition for enhancing the rate of cleavage or formation of such specific bonds, in vivo, by a rate-enhancing antibody to be introduced into an animal which is prepared by a rational design method according to the invention.

It is still a further object of the invention to provide a pharmaceutical for enhancing the rate of cleavage or formation of specific amide, peptide, ester, or glycosidic bonds within biomolecules, in vivo, by an effective amount of an antigen to be introduced into an animal which, by action upon the animals immune system, elicit antibodies which enhance the rate of cleavage or formation of those bonds.

It is still a further object of the invention to activate or deactivate a biological function in an animal by enhancing the rate of cleavage or formation of specific bonds, in vivo, by rational design methods which include identifying the biomolecule which is to be formed or cleaved so that the desired biological activation or deactivation can be achieved and thereafter selecting the appropriate antigen so that the desired rate-enhancing antibody can be elicited.

It is still a further and related object of this invention to provide algorithms and to identify certain amino acid sequences in biomolecules wherein the desired cleavage or formation can be achieved.

It is still a further object of the invention to provide antigens which are analogous to vaccines and which cause the animal's immune system to develop a desired catalytic antibody which then acts upon a biomolecule to enhance the rate of cleavage thereof and to achieve the desired activation or deactivation of a biological function.

It is still a further object of the invention to cause the activation or deactivation of certain biological functions, e.g., the reduction of allergic symptoms, the reduction of infectivity of viruses, the detoxification of lipopolysaccharide, the mitigation of the effects of tumor necrosis factor, and the reduction of hypertension by decreasing the amount of human renin in vivo.

It is still a further object of the invention to cause the activation of a prodrug.

The invention thus provides the use of an effective amount of a rate-enhancing antibody in the manufacture of a pharmaceutical composition for enhancing the rate of cleavage or formation of a specific amide, peptide, ester or glycosidic bond within a biomolecule *in vivo*, said antibody having been produced by the method of: (a) selecting the specific amide, peptide, ester or glycosidic bond to be cleaved or formed; (b) selecting an antigen comprising an analog of said biomolecule and containing an analog of said bond to be cleaved or formed; (c) exposing cells capable of producing antibodies to said antigen and thereby generating antibody producing cells; (d) hybridizing said antibody producing cells with myeloma cells and thereby generating a plurality of hybridoma cells each producing monoclonal antibodies; and (e) screening said plurality of monoclonal antibodies to identify a monoclonal antibody which enhances the cleavage or formation of said amide, peptide, ester or glycosidic bond.

The invention also provides the use in the manufacture of a pharmaceutical for enhancing the rate of cleavage or formation of a specific amide, peptide, ester or glycosidic bond within a biomolecule *in vivo*, of an effective amount of an antigen capable of eliciting antibodies in an animal which enhance the rate of cleavage or formation of said bond, said antibody having been produced by the method of: (a) selecting the specific amide, peptide, ester or glycosidic bond to be cleaved or formed; (b) selecting an antigen comprising an analog of said biomolecule and containing an analog of said bond to be cleaved or formed; (c) exposing cells capable of producing antibodies to said antigen and thereby generating antibody producing cells; (d) hybridizing said antibody producing cells with myeloma cells and thereby generating a plurality of hybridoma cells each producing monoclonal antibodies; and (e) screening said plurality of monoclonal antibodies to identify a monoclonal antibody which enhances the cleavage or formation of said amide, peptide, ester or glycosidic bond.

The invention further provides the use of an effective amount of a rate-enhancing antibody in the manufacture of a pharmaceutical composition for activating or deactivating a biological function in an animal by enhancing the rate of cleavage or formation of a specific amide, peptide, ester or glycosidic bond within a biomolecule *in vivo,* said antibody having been produced by the method of: (a) identifying a biomolecule whose formation or cleavage leads to the activation or deactivation of the desired biological function, said biomolecule containing an accessible bond to be cleaved or formed; (b) selecting an antigen which comprises an analog of the said biomolecule containing an analog of said bond to be cleaved or formed; (c) exposing cells capable of producing antibodies to said antigen and thereby generating antibody producing cells; (d) hybridizing said antibody producing cells with myeloma cells and thereby generating a plurality of hybridoma cells each producing monoclonal antibodies; and (e) screening said plurality of monoclonal antibodies to identify a monoclonal antibody which enhances the cleavage or formation of said amide, peptide, ester or glycosidic bond.

The invention yet further provides the use in the manufacture of a pharmaceutical for activating or deactivating a biological function in an animal by enhancing the rate of cleavage orformation of a specific amide, peptide, ester or glycosidic bond within a biomolecule *in vivo*, of an effective amount of an antigen capable of eliciting antibodies in said animal which enhances the rate of cleavage or formation of said bond, said antigen having been produced by the method of: (a) identifying a biomolecule whose formation or cleavage leads to the activation or deactivation of the desired biological function, said biomolecule containing an accessible or surface-located bond to be cleaved or formed; and (b) selecting an antigen which comprises an analog of the said biomolecule containing an analog of said bond to be cleaved or formed.

### BRIEF DESCRIPTION OF THE DRAWINGS

The invention, as well as other objects, features and advantages thereof will be understood more clearly and fully from the following detailed description, when read with reference to the accompanying drawings, in which:
Fig. 1 shows the dose dependent inhibition by clone AHIV 1.3 of HIV-I p 24 gag production in infected H9 cells; and
Fig. 2 shows the dose dependent inhibition by clones AHIV 1.3, AHIV 1.6, and AHIV 2.0 of HIV-1-induced cell-fusion.

### DETAILED DESCRIPTION OF THE INVENTION

### Definition of Terms

In its broadest sense, the term "antigen" is defined as a molecule which induces the formation of an antibody. As used herein, the term "antigen" means a molecule which is inherently immunogenic, a hapten according to the invention or an immunogen which comprises a hapten according to the invention coupled to a carrier molecule by a suitable coupling moiety. Carrier molecules include, for example, keyhole limpet hemocyanin (KLH), thyroglobulin, chicken immunoglobulin, ovalbumin, bovine serum albumin (BSA), T-helper peptides, etc. "Coupling moieties" as used herein refer to biotechnological cross-linking reagents well known in the art (e.g., commercially available from Pierce, Rockford, Illinois) and include, for example, Trout's reagent, dissuccinyl suberate, etc.

The term "antibody" includes whole immunoglobulins and fragments thereof which contain the binding site for the antigen.

The term "transition state analog" as used herein refers to an array of atoms which is designed to approximate or "mimic" the configuration of an amide bond or an ester bond as such bonds exist in a hydrolytic transition state.

The term "dipeptide analog" as used herein refers to a structure which comprises a transition state analog or strained ground state analog or elements of both having side chains of two amino acids which are in positions analogous to those of the dipeptide being mimicked. In other words, in a dipeptide analog, the normal amide bond (i.e., -CO-NH-) between the two amino acids has been replaced by an array of atoms as defined above. Additional amino acid residues may be incorporated to surround the dipeptide analog to form a polypeptide. Thus, the dipeptide analog replaces the peptide bond "targeted" for cleavage in the substrate molecule. The moieties surrounding the dipeptide analog contain peptide bond linkages which can be altered such that the naturally occurring C=O group is replaced by NH, O, S, CH₂, CF₂ or C=S and/or the naturally occurring NH group is replaced by O, S, CH₂, CF₂, C=O or C=S. For example, the moieties can be retropeptides in which the C=O and NH groups of the amide bonds are interchanged.

The terms "some or all" refer to a portion of the target molecule including at least the amide, ester or glycosidic bond to be cleaved or all of the target molecule. For example, in an embodiment of the invention, haptens designed for the purpose of eliciting antibodies to catalyze the cleavage of a specific peptide bond in a protein molecule comprising a polypeptide of many amino acid residues, the dipeptide analog corresponding to the target peptide bond need only be surrounded by not more than about eight amino acid residues. However, if the target molecule is a relatively short peptide, it is advantageous to surround the peptide bond analog with all the amino acid residues of the target molecule. One of ordinary skill in the art will realize that the desired specificity, the nature of the target molecule and other factors will dictate the ideal number of amino acid residues needed to surround the dipeptide analog.

The term "substantially corresponds" refers to moieties which are similar in charge and/or size to moieties in the antigen containing the analog of the bond to be cleaved. Preferably, the moieties are identical in size and charge, although such identity is not necessary for the hapten of the invention. Haptens may contain one or more asymmetric centers and therefore exist in enantiomeric and/or diastereomeric forms. In general, haptens are obtained in the form of racemates or mixtures of diastereomers. If desired, techniques well known in the art for the separation of the mixtures into sterically homogeneous constituents may be used. Preparation of the optical isomers in a pure state is also possible by using sterically homogeneous starting materials.

The term "hapten" as used herein is defined as a molecule which can act as an epitope. Haptens incorporate an amide, peptide, ester, or glycosidic bond to be cleaved or formed.

Physiologically acceptable salts include salts of mineral acids, for example, hydrochloric acid, sulfuric acid, nitric acid and the like, salts of monobasic carboxylic acids such as, for example, acetic acid, propionic acid and the like, salts of dibasic carboxylic acids such as, for example, maleic acid, fumaric acid and the like, and salts of tribasic carboxylic acids such as, for example, citric acid and the like.

The term "naturally occurring amino acid" as used herein includes the twenty essential alpha-amino acids and other alpha-amino acids which may or may not be found in proteins. These amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, 4-hydroxyproline, 5-hydroxylysine, epsilon-N-methyllysine, 3-methylhistidine, beta-alanine, gamma-aminobutyric acid, homocysteine, homoserine, citrulline, ornithine, canavanine, djenkolic acid and beta-cyanoalanine. An amino acid consists of a carbon atom to which is bonded an amino group, a carboxyl group, a hydrogen atom and a distinctive group referred to as a "side chain." The term "analog of said side chain" as used herein is defined as a side chain of a naturally occurring amino acid in which one or more moieties of the naturally occurring side chain is replaced by one or more different moieties which substantially corresponds to the naturally occurring moiety. Those side chains containing a hydroxy group can be glycosylated, phosphorylated, sulphonylated or protected by a hydroxy protecting group. The hydroxy group of any of the side chains may be protected by any number of suitable hydroxy protecting groups well known in the art. These include, for example, a tertiary butyl group.

The term "terminal amino protecting group" means any group capable of protecting the terminal amino moiety of a peptide or amino acid. Therefore, terminal amino protecting groups include acetyl, succinyl, biphenylcarbonyl, benzoyl, t-butyloxycarbonyl, carbobenzyloxy, tosyl, dansyl, isovaleryl, phthalyl, 1-adamantanesulphonyl, acetimido, benzimido, amidino, carbamyl and the functional equivalents thereof.

The term "terminal carboxyl protecting group" means any group capable of protecting the terminal carboxyl moiety of a peptide or amino acid. Terminal carboxyl protecting groups include (C₁-C₉)alkyl, phenyl, substituted methyl esters such as methoxymethyl and phenacyl esters, 2- substituted ethyl esters such as cyclohexyl and allyl, substituted benzyl esters such as para-methoxybenzyl and para-bromobenzyl, amides such as piperidinyl and hydrazide and functional equivalents thereof.

The term "biomolecule" is defined as any molecule which affects a biological system in vivo or in vitro. Biomolecules may be synthesized by cells or chemically synthesized. Examples of biomolecules include proteins, glycoproteins, peptides, steroids, nucleic acids, and oligo- or polysaccharides. Also included are synthetic organic analogs of peptides, steroids, nucleic acids, etc. Pharmaceutically active compounds such as theophylline, capoten, cyclosporin, etc., are also considered to be biomolecules.

The term "accessible" means capable of combining with the combining site of an antibody.

A catalytic antibody is an antibody which is capable of changing the rate of a chemical reaction, all other conditions (e.g., temperature, reactant/substrate concentration, etc.) being the same, which does not enter into the chemical reaction and therefore is not consumed in the reaction, and which has the capability of converting multiple moles of reactant/substrate per mole of catalytic antibody. From a mechanistic viewpoint, it binds the reactant/substrate, effects the accelerated conversion of the reactant/substrate to the product and then releases the product, changes the rate of the chemical reaction without shifting the position of the equilibrium. The aforementioned definitions are characteristics of ideal catalysts. However, in practice, even the best of catalysts become poisoned or deactivated by contamination in the reaction system or as a result of chemical or physical destruction during the reaction process. For reasons well known in the art, the true operation of a catalyst may be obscured by components of the reaction system or by the condition of the reaction environment.

A stoichiometric antibody is an antibody which enhances the rate of the chemical reaction stoichiometrically, i.e., it enhances the rate of the reaction, but unlike a catalytic antibody, is stoichiometrically consumed during the reaction.

### Identification of Biomolecules and Sequences Therein as Targets for Therapeutic Action

In reported examples of catalytic monoclonal antibodies, the specificity of said antibodies is achieved because the contacted molecule, the substrate, is essentially identical to the immunogen with the exception of the transition state structure, which is present only in the immunogen. It has now been found that this approach can be extended to the design'of an immunogen, or hapten, in order to elicit catalytic antibodies capable of catalyzing a reaction in the substrate when that substrate is part of a larger, more complex biological molecule.

For example, in the design of an oligopeptide hapten, the transition state analog is located somewhere within the sequence which leads to induction of a catalytic monoclonal antibody with a peptidase activity. The specificity of the antibody is determined by the amino acids flanking the transition state analog structure. The antibody so elicited will hydrolyze the peptide specifically, while peptides having similar but not identical sequences or structures would not be hydrolyzed. Such a catalytic antibody will also hydrolyze the peptide when that peptide is part of a protein, provided (a) the peptide sequence in question when present in a native protein is available for binding to the catalytic antibody, i.e., the epitope is surface located, and (b) if the peptide sequence in question is available, it is able to assume the conformation in the protein exhibited by the full oligopeptide in its free, substrate active form.

It has now been found that algorithms that identify peptide, ester, or glycosidic bonds within a biomolecule as "accessible" or "nonaccessible," or as "surface" or "nonsurface" can be used to assist the process of antigen design. Such information is available in various forms and with various levels of precision. First, if the three-dimensional structure of the biomolecule is known, then regions determined to be surface located by inspection of the structure can be identified as possible antigens. Three-dimensional structures of all published protein structures are stored in the Brookhaven database (4) and are readily available, while three-dimensional structures of other biomolecules are stored in the Cambridge database.

If no structure is available, then similar information, though less precise, may be obtained by reference to a computer model or predicted structure of the biomolecule. This is particularly helpful when the biomolecule of interest is part of a family of homologous biomolecules, in which case, "knowledge-based" predictions (5) can be made, thus identifying surface located sequences.

At an even less precise level, algorithms can be employed that produce a hydrophilicity profile for the biomolecule. Regions of the biomolecule that contain a high proportion of hydrophilic or charged moieties will be likely candidates for a surface location.

Certain amino acid sequences are preferred targets when cleavage of the protein chain is the goal. For example, sequences containing the following amino acid combinations, ASN-GLY, ASN-PRO, ASP-GLY, and ASP-PRO, when present in a protein or peptide chain, are a preferred site of a catalytic cleavage. Similarly, sequences containing glutamic acid and glutamine, i.e., GLN-X and GLU-X wherein X is any amino acid, are preferred cleavage sites.

A further alternative for designing cleavage of a protein when no other information is available is to follow the purely random approach in which antibodies are raised to various peptides along the protein sequence until a region of cross-reactivity between antibody and protein is located.

In order to effect therapeutic applications of monoclonal antibody technology, the monoclonal antibody must bind to, and not dissociate from, its antigen. The antigen may be a soluble protein, virus, or other toxic molecule, or may be the surface of a cell. After binding, the antibody-antigen complex either triggers the activation of lytic enzyme cascades or is removed by phagocytic cells.

Catalytic antibodies, on the other hand, operate by a fundamentally different mechanism. Since a catalytic antibody, protease, for example, is able to "cut" the protein at its site of binding, the location and design of the epitope must be such that, on proteolysis and subsequent dissociation of the catalytic antibody from its target, the biological function of the target is irreversibly lost. Thus, a noncatalytic antibody directed against an epitope within the receptor binding region of gp 120 from HIV may inhibit binding of the virus to its CD4 receptor (4). This inhibition will arise because the large bulk of the antibody will prevent the virus and its receptor from coming into contact.

While a catalytic antibody protease directed against the same region of the virus may also lead to loss of receptor interaction, to insure loss of infectivity, a different set of events must ensue. The protease will not remain bound after cutting the virus protein chain and therefore the act of cutting itself must lead to loss of binding to be effective. This may not necessarily happen. For example, cleavage of the particular peptide bond may not lead to sufficient disruption of the three-dimensional structure to destroy receptor binding activity. In contrast, proteolysis of a peptide bond within an epitope distant from the receptor binding region may lead to loss of infectivity by virtue of its destabilizing effect on the protein structure. Therefore, since catalytic antibody proteases do not act via a simple steric blocking effect, a radically different design process for the target sequences must be established. Particular examples of how this process may be implemented are described below.

The methods of the invention can also be used to effect a cleavage that leads to the activation of some biological function. Such reactions include the cleavage of peptide bonds, but may also include ester bonds or glycosidic bonds or other types of bonds.

One example of the cleavage of a biomolecule which leads to the activation of a biological function is the treatment of insulin-dependent diabetes. Patients self-administer insulin by injection. The art has searched for a formulation of insulin whose release into the circulation mimics the pharmacokinetics of the release of natural pancreatic insulin. Insulin exists in the pancreas in a pro-form, proinsulin, whose activity is many orders of magnitude lower than insulin itself. An antibody protease specific for the peptide bond that leads to conversion of proinsulin to insulin can be designed so that its kinetic characteristics allow release of insulin in vivo after injection of proinsulin plus antibody protease. This is an example of prodrug activation where the drug in this instance is a natural protein hormone. Prodrugs may include any therapeutically active molecule which leads to the activation of a biological function. The pro-form may either take advantage of a natural modification of the drug or any suitable synthetic modification thereof. Suitable drug derivatives with low activity (therapeutically beneficial or toxic), which, on modification with a suitable catalytic antibody, are converted to an active form. A particular example of this process is given below.

### Preparation and Use of Rate-Enhancing Antibodies

Rate-enhancing antibodies, i.e., stoichiometric and catalytic rate enhancers, may be elicited through both in vitro and in vivo techniques. The term "elicited" as used herein means elicitation of catalytic antibodies by antigens according to the invention through both in vitro and in vivo techniques. However, the skilled artisan will readily appreciate that when in vitro elicitation is involved, the haptens according to the invention, by themselves, may be used to elicit the catalytic antibodies. When elicitation is achieved through in vivo techniques, it is understood that immunogens comprising haptens complexed to a suitable carrier molecule are used to elicit the catalytic antibodies. The antigen comprises a hapten designed to elicit the appropriate hypervariable binding region in an antibody molecule to express intrinsic binding energy for the transition- state of a chemical reaction, particularly a hydrolytic reaction. Arrangement of amino-acid side chains generated in the combining-site will be appropriate for performing chemical modification of an epitope of interest. Additional improvements in catalytic efficiency can be achieved by site-directed mutagenesis.

Broadly, the method comprises exposing cells capable of producing antibodies to the antigen and thereby generating antibody producing cells; hybridizing the antibody producing cells with myeloma cells and thereby producing a plurality of hybridoma cells each producing monoclonal antibodies; and screening the plurality of monoclonal antibodies to identify a monoclonal antibody which catalyzes the chemical reaction of interest. The monoclonal antibody so identified may then be replicated, again by either in vivo or in vitro techniques, to obtain a quantity sufficient to catalyze the chemical reaction of interest.

The detection of antibodies with the desired catalytic activity and specificity is achieved by screening the hybridomas once they have been elicited. For example, screening may be achieved by high performance liquid chromatography (HPLC) or spectrophotometric methods (ELISA). Catalytic monoclonal antibodies are elicited "in vivo" by modification of the technique disclosed by Koprowski et al. in U.S. Patent No. 4,196,265, issued April 1, 1980, which is hereby incorporated by reference. The details of that process are known in the art. A series of monoclonal antibodies directed to a specific molecule are prepared under suitable conditions. This involves first immunizing BALB/C mice with an appropriate antigen. The antigen comprises a hapten according to the invention bound to a peptide or other carrier molecule.

Antibody-producing lymphocytes are then removed from the spleens of the immunized mice and hybridized with myeloma cells such as SP2/0 cells to produce hybridoma cells. These hybridoma cells are then plated in the wells of microtiter plates. The series of monoclonal antibodies being produced by the hybridoma cells is screened under appropriate conditions to identify monoclonal antibodies which catalyze the desired reaction under appropriate conditions. Alternatively, the medium may be tested for antibodies that bind to the immunogen and the hybridomas producing these antibodies then expanded in tissue culture or grown in vivo. Screening may be conveniently accomplished by treating a standardized solution of the reactant with an aliquot of medium withdrawn from a microtiter well and measuring the presence of the desired product by conventional instrumental methods. This measurement may be readily conducted, for example by spectrophotometric methods or by gas-liquid or high pressure liquid chromatography. By comparison with standardized samples of the desired product or reactant, rates of reaction may be quantified. In this manner, wells containing hybridoma cells producing catalytic monoclonal antibodies are identified. The selected hybridoma cells are then cultured to yield colonies.

These colonies may be further propagated in vitro or in vivo systems. In the latter case, mice such as syngeneic BALB/C mice are inoculated intraperitoneally with the selected hybridoma cells and produce tumors, generally within two or three weeks. These tumors are accompanied by the production of ascites fluid which contains the desired monoclonal antibodies. The monoclonal antibodies are then separately recovered from the ascites fluid by conventional methods such as ultrafiltration, ultracentrifugation, dialysis and immunaffinity chromatography.

The separately recovered monoclonal antibodies are introduced to an animal in vivo under suitable conditions permitting the formation of a complex between the monoclonal antibody and the target molecule. In general, the concentration of the catalytic antibodies used is less than the equivalent concentration of the target molecule and may be in the picomolar range. The antibodies should function under normal physiologic conditions in vivo. The skilled artisan will appreciate that the conditions suitable for complex formation may vary depending on the particular molecule and antibody under consideration.

Antibodies produced by any of the processes described above may be produced via an immortalized cell line, in vitro. A suitable form of the antibody (for example, a "humanized" mouse monoclonal antibody) would then be administered as a therapeutic "drug."

During the course of a chemical reaction, the reactants undergo one or more transitions through structures which are energetically less favorable than either the reactant or product. In molecular terms, these transition states (or intermediate structures) reflect changes in bond lengths and bond angles as well as formations and breakages of bonds. The energy required to achieve a transition state is denoted as the activation energy, which may also be considered as the difference in energy between the energy of the transition state and the energy of the reactants.

Catalysts increase chemical reaction rates by lowering the activation energy of a reaction. Antibodies elicited to a hapten or antigen, which antigens are chosen because, inter alia, they resemble the presumed transition state structure (i.e., a transition state analog, a strained ground state structure or both), can catalyze reactions. The antibody thus produced should stabilize the energy of the transition state relative to reactants and products. This approach has been successfully demonstrated in the generation of several catalytic monoclonal antibodies.

Catalytic antibodies elicited with rationally designed haptens are "site specific" in that they are deliberately designed only to catalyze cleavage of bonds having certain structural conformations at specific sites in a biomolecule. Likewise, these catalytic antibodies are designed only to catalyze the formation of bonds from the termini of moieties having certain structural conformations at those termini. Therefore, rationally designed haptens according to the invention may be used to elicit a site specific catalytic antibody capable of cleaving bonds at specific sites in a biomolecule to produce two or more cleavage products. The same catalytic antibody can then catalyze the formation of bonds wherein those cleavage products having the right structural conformation are joined.

Thus, the haptens are designed to mimic the transition states for a variety of chemical reactions. Preferably, the reactions are the cleavage or formation of a peptide, ester, amide, or glycosidic bond. For example, a hapten as shown below, 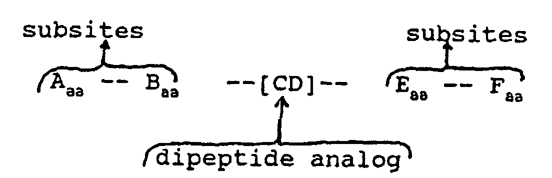 incorporates not only the dipeptide analog [CD] but also sub-site amino acid residues A, B, E, F. These subsite amino acid residues can be part of a cyclic structure as well as a linear structure. The optimum number of sub-site residues is determined by the size of the antibody combining site. It is likely that the only essential criterion for effective binding of antibody to a peptide is that complementarity between the antigen combining site of the antibody and the molecular surface of the binding peptide is maintained with regard to both shape and charge.

The haptens are designed in such a way such that antibodies raised against these haptens can selectively stabilize one or any of the high energy intermediates or transition states in the cleavage or formation of an amide, peptide, ester or glycosidic bond. These haptens fall into three general classes: one, those in which the hybridization of the atom corresponding to the carbonyl carbon of the scissile bond of the amide or ester bond is converted from sp² to sp³ hybridization; two, those in which any of the atoms corresponding to the amide, ester or glycosidic bond is replaced by a different atom; and three, those in which the atoms corresponding to the amide, ester or glycosidic bond are part of a monocyclic or bicyclic system.

Peptide sequences containing dipeptide analogs at the bond that is required to be hydrolyzed by the catalytic antibodies of one aspect of the present invention define a sequence that the catalytic antibody will hydrolyze in a native protein. The binding energy of the antibody is distributed in such a way as to allow both sequence specific recognition and chemical reactivity with the native protein or peptide of interest.

It has been reported that it is not necessary to prepare peptides longer than eight amino-acid residues (octapeptides) to demonstrate all continuous epitopes (5). It has also been demonstrated that antibodies bind to peptides in a reproducible manner (6). It has also been established that optical isomerism of the amino acids used has a powerful influence on the strength and specificity of antibody binding by dipeptides. Consequently, the importance of L and D amino acid residues in the immunizing antigen will have a profound effect on the chirality of the antibody combining site generated. In generating catalytic antibodies according to one aspect of the invention with predetermined specificity for particular sequential (continuous) or assembled epitopes in a native protein, the relationship between measurable properties of a protein and its immunogenic sites are important (7). With the ready availability of protein sequences, the most widely used algorithm is based on the likelihood of finding a sequential epitope at the site of a local maximum in the hydrophilicity profile (8). Surface accessibility profiles (9) and protein flexibility (10) also provide information on the antigen sites in a native protein sequence. With knowledge of these sites and the importance of these epitopes in receptor mediated interactions or other disease associated mechanisms, peptide haptens having dipeptide analogs within these important "bioactive" epitopes can be designed in accordance with the invention. The catalytic antibodies elicited with these haptens can then be utilized, for example, to digest epitopes on viral proteins or tumor derived growth factors or other peptides involved in life-threatening situations (e.g., tumor necrosis factor in bacterial sepsis, etc.).

Thus, the haptens are rationally designed from knowledge of mechanistic features of enzyme catalysis and provide suitable templates for generating antibody combining sites endowed with catalytic properties. Consequently, they incorporate all the necessary features to provide for antibodies capable of molecular recognition and catalytic function. Thus, a cyclic carbohydrate hapten of formula [1] would provide a good mimic of the proposed transition state [2] (11) for hydrolysis of a glycosidic bond in a typical O-linked glycoside [3], as illustrated below:

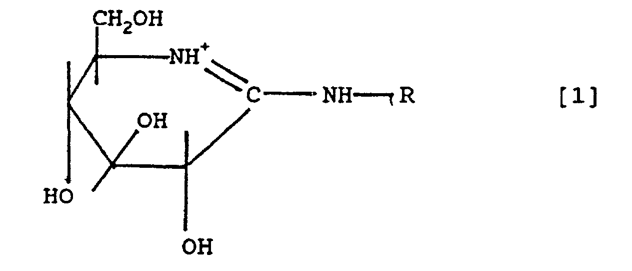

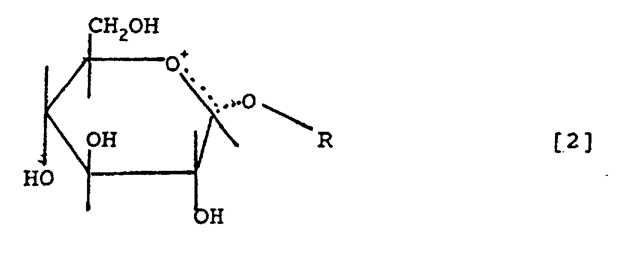

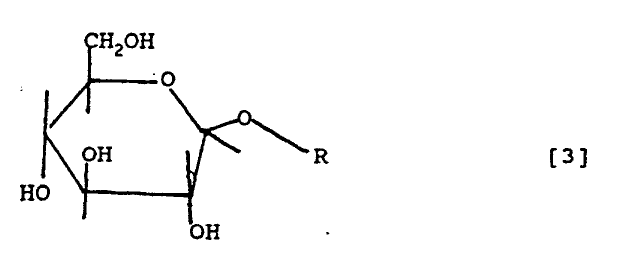

Catalytic antibodies have site-specific proteolysis capabilities in, e.g., the immunotherapy of viral infection. Viruses utilize their external coat proteins to attach to cellular receptors and invade the cell after attachment. For example, human immunodeficiency virus (HIV) uses a portion of the gp120 protein at its surface to attach to CD4 receptors on lymphocytes. The sequence for this cell attachment has been mapped to a region on the viral protein. With this information, antibodies can be generated by the methodology described in this invention to bind to this peptide sequence and cleave it in a site-specific manner. However, such antibodies preferably bind to the "native" sequence in the protein as opposed to a linear sequence (which would occur in a denatured protein). Thus, the antigenic determinants or epitopes in the "native" protein are often conformational (i.e., three-dimensional) rather than random linear arrangements. Here again, knowledge of epitopes on the protein is important in the design of antibodies having paratopes that can induce modifications of such epitopes. Therefore, haptens may be designed to have the same structural features of the epitopes, rather than random conformations. These structural features can be adopted by simple linear peptides, the lowest energy conformer being the preferred structure in solution. Secondary structural features may be introduced by crosslinking of amino-acid side chains or the use of -turn mimetics. Conformationally constrained haptens incorporating structures which are compatible with the epitope in the native protein may be essential for inducing the correct motif within the tertiary structure of the catalytic antibody hypervariable binding region. The advantages of conformationally constrained haptens are that they mimic the native structure in the protein and tend to mimic regions of the protein which are susceptible to cleavage.

Oligopeptides having variable lengths with sequences from the receptor-binding regions of viruses which employ a specific cellular receptor for penetration of the host cell and having a transition state analog dipeptide isostere in a critical region of the sequence induce on immunization, optionally after coupling to a suitable carrier protein, catalytic antibodies that cleave the viral coat protein and prevent virus penetrating the cell. The dimensional structure of Rhino 14 and Polio 1 virus particles has been charted by X-ray scattering. Regions have been identified which are binding sites to cellular receptors. The region of the human immunodeficiency virus type 1 (HIV I) critical for interaction with the CD4 receptor on T-lymphocytes has been located and mapped to sequences in the gp120 coat protein. Thus, in one embodiment of the invention, oligopeptides are used which contain partial sequences from the envelope proteins of viruses critical for host cell attachment and a transition-state dipeptide isostere selected from the haptens according to the invention. The resultant peptide analogs are used to induce catalytic antibodies that inactivate viruses by proteolysing segments (epitopes) of the viral coat protein critical for infectivity. Preferably, oligopeptides are used having sequences from the receptor binding region of retroviruses, e.g., HIV I, HIV II and picorna viruses, e.g., Rhino 14, viral polypeptides, inflammatory proteins, anaphylactic proteins, lymphokines, cytakines and other polypeptide mediators of host infection or toxic syndromes.

Catalytic antibodies elicited with the antigens of the invention may be useful in the treatment of autoimmune disease, cancer and thrombolytic disease. The catalytic antibodies may also be useful for treatment of cardiovascular disease eliminating high density lipoproteins and for the detoxification of bacterial endotoxins.

### Preparation and Use of Antigens as Vaccines

It has also been found that advantage may be gained by exploiting the body's own ability to produce antibodies in vivo, in response to a certain antigenic stimulus. Processes are well known where the immune response to a particular pathogen can be primed by administration of either an inactive form of the pathogen or a peptide, antibodies against which cross-react with the pathogen. When the real pathogen is encountered, antibody-producing B-cells having the correct antibody specificity are already present. Such priming agents are commonly called vaccines ( ). A similar process may be envisaged for induction of a catalytic antibody. In a process whereby the particular catalytic antibody to be induced is part of a systematic procedure, a hapten is designed to include a transition state analog for cleavage at a particular location. Immunization with this hapten would then activate certain B-cells, some of which would produce antibodies with catalytic activity. On exposure to the "normal" antigen (substrate), the primed cells in the animal would then respond giving rise to a catalytic antibody in vivo, thus obviating the need for parenteral administration. These "catalytic" vaccines might have very general applications in activation of catalytic antibodies having activities against viruses and other pathogens, toxic agents, drugs of abuse, naturally occurring proteins, therapeutically useful prodrugs.

The invention will be more fully described and understood with reference to the following illustrative examples.

### Example 1

### Methodology For The Production, Screening And Isolation Of Monoclonal Catalytic Antibodies

### That Cleave The "Flap" Region Of Human Renin

The inhibition of renin, an aspartic proteinase whose action initiates the renin-angiotensin cascade has been the object of intense investigation in recent years. The potential for treatment of hypertension through the inhibition of renin has resulted in the synthesis of a variety of potent renin inhibitors based on the peptide sequence of the natural substrate angiotensinogen. An alternative approach is the use of a proteolytic antibody to renin.

It has been reported that the flap region of human renin is a hairpin with a loop region of four amino acid residues leading to the carbonyl group of Tyr 83 interacting with the amino groups of Thr 85 and Gly 86 (12). The cyclic decapeptide 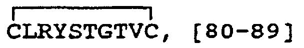
CLRYSTGTVC, [80-89]
 human renin, was found to adopt this same hairpin structure.

In this example, monoclonal antibodies are elicited with a difluoroketone containing immunogen according to the invention. The monoclonal antibodies will catalyze the cleavage of the peptide sequence 
 81              85  86              90
Leu-Arg-Tyr-Ser-Thr-Gly-Thr-Val-Ser-Gly
 in the human renin molecule between residues 85 and 86. Cleavage causes disruption of the catalytic machinery of the enzyme since residues 85 and 86 constitute the "flap" region which holds the substrate in the catalytic site (13). It has previously been demonstrated that rabbit polyclonal antiserum elicited to a synthetic peptide fragment (sequence 81-90) could inhibit human renin activity by 40% as measured by its reaction with synthetic human tetradecapeptide substrate (14).

In order to raise a catalytic antibody to this flap region of human renin, the cyclic peptide hapten CLRYST(TS)GTVC, where (TS) represents the transition state analog, is synthesized. In this example, the synthesis of the cyclic peptide hapten 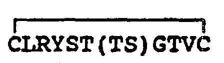
CLRYST(TS)GTVC
 follows the solid-phase approach (15) wherein ST(TS)G corresponds to a transition state analog tripeptide according to the invention, and is incorporated into the assembled peptide through its anhydride (15). The. completed peptide is fully deprotected and cleaved from the solid support using trifluoroacetic acid.

Cyclization of the peptide is achieved by allowing the peptide to stand at low concentration for one hour in an aqueous solution (pH 8) in order to generate a disulfide bridge between the two terminal cysteine residues. The N-terminal amino group of the peptide allows it to be attached to a carrier protein for immunization of mice.

### A. Preparation of the Immunogen

### 1. Peptide Synthesis

The peptide hapten is synthesized by the solid phase technique using the polyamide-Kieselguhr composite resin (14). The side chain protecting groups are the following: O-tert-butyl (tyrosine, glutamic acid, serine, threonine); N-4-methoxy-2,3,6-trimethyl benzenesulphonyl (arginine); and S-trityl (cysteine). The temporary protection of the N function is by fluorenylmethoxycarbonyl which is removed in 10 minutes with piperidine/DMF : 20/80. The coupling reactions are carried out using FMOC- amino acid anhydrides (14). The protected peptidyl-resin is fully deprotected by treatment with trifluoroacetic acid/thioanisole/m-cresol/thiophenol/ethanedithiol solution: 90/2/2/2/4 for 3 hrs. After filtration the filtrate is concentrated under vacuum to a small volume. Ether is added to give a precipitate of the peptide. The ethereal supernatant is removed and the peptidic precipitate is washed twice with ether to yield the peptide hapten 
O
||
Cys-Leu-Arg-Tyr-[Ser-Thr-C-CF2-Gly]-Thr-Val-Cys
 wherein the bracketed moiety is a difluoroketone transition state analog tripeptide isostere.

### 2. Cyclization of Peptide Hapten

The peptide hapten is cyclized to obtain the "β-hairpin" conformation by forming a disulfide bond between the two terminal cysteine residues. The disulfide bond is completed in one hour by air oxidation of an aqueous solution (pH 8) at a concentration of 0.3 mg of peptide per milliliter. The oxidized product is then removed by lyophilization and purified by HPLC.

### 3. Conjugation of the Hapten to the Carrier Molecule

The cyclic peptide hapten 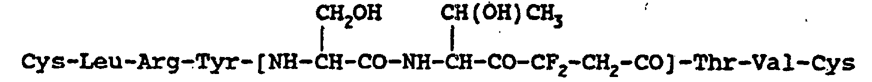 is conjugated to keyhole limpet hemocyanin (KLH) using glutaraldehyde. Coupling efficiency is 50-80% as estimated by binding of a trace amount of I125 peptide added to the reaction mixture.

### B. Preparation and Screening of Monoclonal Antibodies

KLH conjugated peptide (50 µg) in complete Freund's adjuvant is injected into BALB/c mice. Hybridoma fusions are carried out by standard methods using SP2/0 myeloma as the fusion partner. Polyclonal antiserum response and hybridoma secreting cells resulting from the fusion are screened for binding activity by ELISA.

Wells of plastic microtiter plates (Falcon 3915 Probind, Becton Dickinson Labware CA, USA) are coated with 50 µL of peptide (5 µg/mL) in Tris-HCl buffer (0.1 M, pH 9.6).

Plates are first incubated for 30 minutes at 37°C and then overnight at room temperature. After washing three times with Tween- containing phosphate-buffered saline (PBS-Tween 0.1%, pH 7.4), 50 µL of serial dilutions of antisera in PBS-BSA 1% pH 7.4 are added in peptide-coated duplicate wells and incubated for 2 hours at 37°C. Plates are washed three times again with PBS-Tween 0.1% and wells are then treated with 50µL of alkaline phosphatase-labelled goat anti-mouse IgG diluted 1:500 (Sigma, MO, USA). Incubation is carried out for 1 hour at 37°C.

Additional extensive washing with PBS-Tween 0.1% is followed by incubation with 150 µL of alkaline phosphatase substrate (2 tablets/10 ml of Sigma 104-105) dissolved in 0.1 M glycine-NaOH buffer (pH 10.4) containing MgCl₂ and ZnCl₂, 1mM. The enzymatic reaction is allowed to proceed for 2 hours at 37°C and stopped by addition of 50 µL of Na₂CO₃ (1.5 M).

Absorbance is read at 405 nm in a Titerteck Multiskan ELISA reader (Flow laboratories). Titer expression is determined by multiplying the optical density by the maximal dilution giving an absorbance three times as high as the negative control (consisting of pooled normal mouse sera diluted 1:100).

Hybridomas giving a positive reaction in this screening assay are chosen for further study. IgG is purified from ascites fluid by HPLC with a Bakerbond ABx HPLC column.

### C. Catalysis of Peptide Cleavage by Catalytic Antibody Specific for the "Flap" Region of Human Renin

The decapeptide substrate H2N-Leu-Arg-Tyr-Ser-Thr-Gly-Thr-Val-Ser-Gly-CO2H (2.7 uM) is incubated with the catalytic antibodies produced by the procedure outlined above and the reaction monitored by reverse phase HPLC analysis of the mixture. The reaction is carried out at pH values optimal for high Kcat by the catalytic antibody (optimum pH is determined employing the chromogenic p-nitroanilide substrate 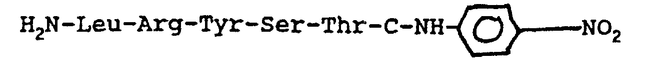 H2N-Leu-Arg-Tyr-Ser-Thr-C-NH--NO2 or the fluorescent coumarin substrate 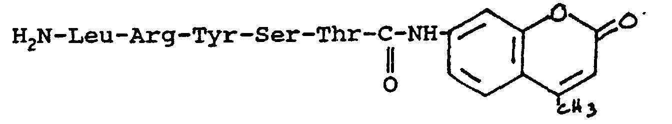
H2N-Leu-Arg-Tyr-Ser-Thr-C-NH-
O
CH3
 and taking into account the binding energy of the catalytic antibody for the residues on the C-terminal side of the cleavage site with change in pH).

Antibodies that show the best Kcat values for cleavage of the decapeptide substrate are tested for their ability to inhibit human renin.

### D. Binding of Anti-Transition-State Analog Antibodies to Human Renin and Inhibition of its Enzymatic Activity by Cleavage of Residues 85-86 in the "Flap" Region

Inhibition of plasma renin activity -- the ability of the catalytic antibodies to inhibit renin activity -- is tested on a pool of human plasma having a high renin activity (40 ng of angiotensin I/h/mL). Plasma (25 ul) is pre-incubated with 100 ul of the catalytic antibody in PBS (pH 7.5) containing 1% EDTA (final volume 0.2 mL) for various periods of time. Next, an excess of plasma renin substrate (200 pmol) is added in order to ensure zero-order behavior, and the mixture is incubated for 30 min. at 37°C in PBS (pH 5.7). The final dilution of the catalytic antibody is 1:5 and 1:50. The angiotensin I generated is measured. by radioimmunoassay (17). A blank is included using the same dilutions of the corresponding abzymes. The amount of angiotensin I generated is less than that observed with intact renin, thus indicating cleavage of residues 85-86 in the "flap" region and inhibition.

### Example 2

### In vivo Preparation of Immunogenic Traasition-State Analog Containing Peptide from HIV gp120 Coat Protein

The octapeptide sequence -Ala-Ser-Thr-Thr-Thr-Asn-Tyr-Thr- from HIV gp120 coat protein is important for virus interaction with the OKT4 antigen of T4 helper/inducer cells. Synthetic peptides identical or very similar in sequence to this octapeptide strongly inhibit attachment of HIV to the antigen receptor (18). Computer assisted searches have demonstrated homology to a peptide found in the envelope region of the Epstein-Barr virus. In addition, strong homologies exist between the HIV octapeptide and peptides which occur in human lymphoadenopathy virus (LAV) and in human T-cell leukemia virus (HTLV-IIIb) isolates. An additional homology exists between the HIV peptide and a sequence comprising residues 19-26 of bovine pancreatic ribonuclease A (RNase A). This sequence contains the exposed subtilisin cleavage sites of RNase A between residues 20 and 21 and 21 and 22.

Peptide haptens according to the invention are: (1) -Ala-[Ser-A-Thr]-Thr-Thr-Asn-Tyr-Cys (2) -Ala-Ser-[Thr-A-Thr]-Thr-Asn-Tyr-Cys (3)   -Ala-Ser-Thr-[Thr-A-Thr]-Asn-Tyr-Cys (4)   -Ala-Ser-Thr-Thr-[Thr-A-Asn]-Tyr-Cys The transition state dipeptide isosteres (indicated in brackets) wherein A represents an analog of the amide bond to be cleaved are incorporated into the peptides as described in Example 1. Each peptide hapten is coupled with keyhole limpet hemocyanin (KLH) carrier protein through the terminal cysteine residue utilizing the cross-linker m-maleimidobenzoyl-N- hydroxysuccinimide ester (20).

### B. Preparation of Monoclonal Antibodies

BALB/C mice are immunized with the KLH-peptide analog conjugates emulsified in complete Freund's adjuvant. A blood sample is obtained from each mouse and the serum separated by centrifugation and stored at 4°C. Sera obtained in this way are screened for binding activity to the original transition-state analog immunogen by standard ELISA procedures. Antibody producing mice immunized as described above and assayed for reactivity with the transition state analog peptide immunogens are sacrificed and their spleens removed and hybridoma cells are prepared using SP2/0 myeloma cells as described in Example 1B above.

### C. Screening Hybridoma Cells Producing Catalytic Monoclonal Antibodies

Screening for binding of antibodies to respective transition state analog-containing peptides is performed essentially as described in Example 1 above. Hybridomas secreting monoclonal antibodies and giving a positive binding reaction are chosen for further study. IgG is purified from ascites fluid by HPLC with Bakerbond ABxHPLC.

### Example 3

### In Vitro Elicitation of Catalytic Monoclonal Antibodies Against A Viral Epitope That Selectively Inhibits Human Immunodeficiency Virus (HIV)

### A. Preparation of the Immunogen

The dipeptide transition state isostere, 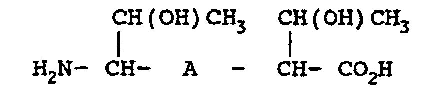 wherein A is an analog of the amide bond to be cleaved, is incorporated into a peptide as described in Example 1 to give the hapten 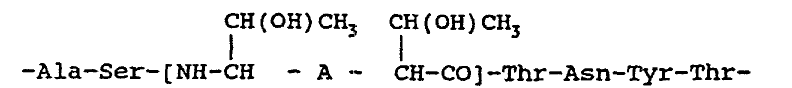 The resulting hapten is designed to mimic a portion of the HIV gp120 coat protein. The synthetic peptide is used in an in vitro immunization procedure using a modification of a literature procedure (21). Spleen cells are cultured at 106 cell/ml in a medium consisting of 50% fresh Eagle's MEM with 20% fetal bovine serum 5 x 10⁻⁵M β-mercaptoethanol, 2mM glutamine and 50% conditioned medium from BALB/c mouse thymocytes. To furnish conditioned medium, thymocytes are cultured at 3-5 x 10⁶ cells/mL in the same Eagles MEM medium as above. After 48-72 hours the medium is removed, sterilized by filtration and used immediately for in vitro activation. The antigen is added to the spleen cell culture medium at concentrations equivalent to approximately 1 µg peptide/ml. Spleen cells are cultured in the presence of antigen for four days without a change in the medium and are later hybridized.

Hybridizations are carried out with the mouse plasmacytoma cell line 45 6TGL.7 (22) obtained from the Cell Distribution Center of the Salk Institute. Spleen cells (either freshly isolated or from in vitro activation cultures) and plasmacytoma cells are washed twice in serin-free Eagles MEM, then fused using PEG 1000 as previously described in the literature. Hybrids are selected by treatment of the cultures with HAT. Hybrid cells which produce antibody reactive with the peptide transition state analog (as judged by an ELISA assay) are cloned and re-cloned by limiting dilution in conditioned medium from parental plasmacytoma cells.

Screening for binding of antibodies to respective transition state analog-containing peptides is performed essentially as described in Example 6 above. Hybridomas secreting monoclonal antibodies giving a positive binding reaction are chosen for further study. IgG is purified from ascites fluid by HPLC with Bakerbond ABₓHPLC. One of ordinary skill in the art will realize that these antibodies can be tested against the peptide not containing the transition state analog.

The virus replication assay is carried out essentially as described in the literature (25), except that cultures are propagated in microtube wells containing 200 µL. Graded concentrations of purified antibodies obtained from the in vitro immunization procedure or buffer alone, each in 25 µL, are preincubated for 1 hr. at 37°C in 5% CO2 with 50 TCID50 HTLV-IIIB in 25 µL. Following preincubation, H9 cells (1 x 10⁵ cells in 150 µL RPM1-040 supplemented with 20% heat-inactivated FCS) are added to the wells, yielding final antibody concentrations ranging from 0.1 µgm1-1 to 10 µgm1⁻¹. Microtiter plates are incubated at 37°C in 5% CO2 for 14 days. Cells are fed by exchanging 100 µL cell-free supernatant fluid on days 3, 7 and 10 with fresh medium, and no further antibody is added during this period. Cell-free supernatant fluid (100 µL) is analyzed for p24 antigen by RIA (Dupont, NEK-040).

The C8166 fusion assay is described in the literature. Three monoclonal antibodies (clone AHIV1.6; AHIV1.3 and AHIV2.0) are tested in 2 hr. assays. H9 cells (1 x 10⁴) infected chronically with HTLV-IIIB are preincubated with varying concentrations of antibody in 150 µL medium in 96-well plates. All assays are done in triplicate. After 1 hr. incubation at 37°C in 5% CO2, 3 x 10⁴ C8166 cells (HTLV-I transformed umbilical cord lymphocytes) in 50 µL are added to the wells. Final well concentrations of antibodies are 41 µg ml⁻¹ and 5 µg m⁻¹. Preincubation with OKT4A (Ortho Diagnostics) at 25 µg ml⁻¹ served as a control. After the plates are incubated for 2 hrs. at 37°C in 5% CO2, syncytia (ballooning cytoplasm greater than three lymphocyte cell diameters) are counted. To prevent bias during counting, samples are coded.

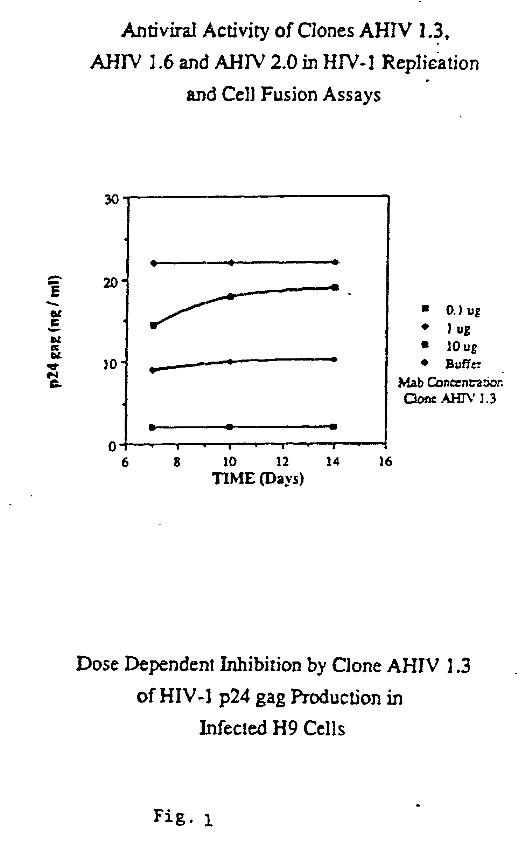

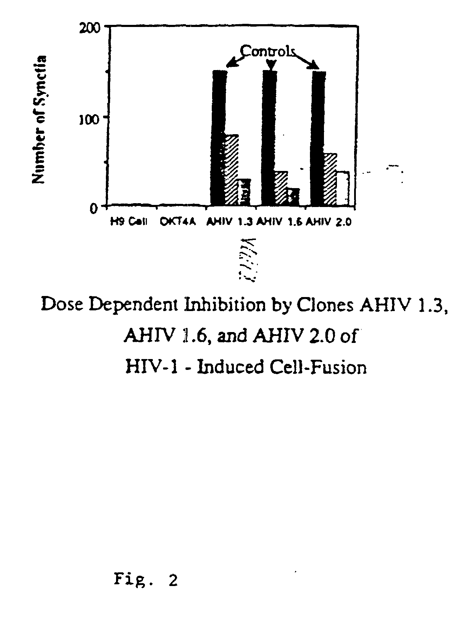

The antiviral activity of clones AHIV 1.3, AHIV 1.6 and AHIV 2.0 is examined in HIV-I replication and cell fusion assays. Fig. 1 shows the dose dependent inhibition by clone AHIV 1.3 of HIV-1 p. 24 gag production in infected H9 cells. Fig. 2 shows the dose dependent inhibition by clones AHIV 1.3, AHIV 1.6 and AHIV 2.0 of HIV-induced cell fusion.

The catalytic monoclonal antibodies elicited by in vitro immunization with the transition state dipeptide isostere analog incorporated into the exposed peptide sequence of HIV gp120 can prevent infection by HIV virus by causing rupture of an important region of the viral coprotein involved in binding to the CD4 receptor on lymphocytes. The catalytic monoclonal antibodies break the peptide bond in the chosen sequence (i.e., between the first two threonine residues from the left of the octapeptide shown in Example 2) in a manner analogous to the action of proteolytic enzymes. The mechanism of the antibody catalyzed hydrolysis of the octapeptide does not involve a metal ion and consequently may involve either a nucleophile in the active site of the antibody or nucleophilic addition of water activated by amino acid residues in the antibody combining site.

### Example 4

### Production of Abzyme Proteases Targeted to Tumor Necrosis Factor

Tumor necrosis factor (TNF) is a cytokine secreted by activated macrophages. TNF has been shown to mediate a variety of biological effects including endotoxin-induced shock, suppression of lipoprotein lipase (LPL) activity in preadipocytes, stimulation of collagenase activity and prostaglandin E2 production by synovial cells, stimulation of interleukin 1 production, and induction of cachexia in nude mice. TNF-specific cell-surface receptors are present on several types of cells. The binding of TNF to these receptors is believed necessary for induction of the biological effects of TNF. It has been shown that antibodies against amino acids 1-15 of hTNF block its binding to cell-surface receptors (Socher et al., Proc. Natl. Acad. Sci. USA, 1987 84, 8829-8833). It is also known that the N-terminal eight amino acids of hTNF are not required for receptor recognition. Consequently, the critical region for receptor binding may involve residues 9-15. The formula below shows the N-terminal 25 amino acids of TNF, the critical residues 9-15(*) and a metastable site NP, Asn-Pro. 
VRSSSRTPSDKPVAHVVANPQAEGQ
         *******   ++
 Synthesis of the peptide analog containing the transition-state dipeptide isostere is performed essentially as previously described in Example 1. Immunogen preparation, immunization, and screening for catalytic antibodies is performed essentially as described except that a bioassay is employed to determine TNF abzyme proteolysis and inactivation.

### Tumor Necrosis Factor Cell Lysis Assay

Murine L-929 fibroblast cells (30,000 per well) are cultured in 96-well tissue culture plates in the presence of 1 µg/ml actinomycin D. Serial dilutions of TNF before and after treatment with the catalytic antibody are added to the wells and incubated for 18h. The culture medium is then removed and the cells stained with a 0.5% crystal violet solution in 25% methanol. The absorbance at 540 nm is measured on a Biotek ELISA microplate reader. The cells with medium alone are considered to have 0% lysis and the cells treated with 3M guanidine-HCL are considered to be completely lysed. One unit of TNF is defined as the amount required to give 50% cell lysis.

### Example 5

### Production of abzyme processes targeted to HIV polypeptides

The primary event in the infection of cells by HIV is the interaction between the viral envelope glycoprotein, gp 120, and its cellular receptor, CD4. Monoclonal antibodies have been produced that block the interaction between gp 120 and CD4. The gp 120 epitope has been shown to be contained within the amino acids 397-439. Deletion of 12 amino acids from this region by in vitro mutagenesis leads to a complete loss of binding and a single amino acid substitution in this region results in significantly decreased binding. The epitope 397-439 has the sequence:

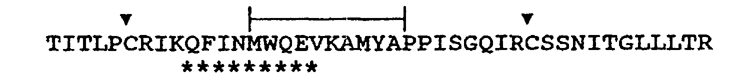
▼ ▼
TITLPCRIKQFINMWQEVKAMYAPPISGQIRCSSNITGLLLTR
*********

The sequence also shows a tryptic peptide from residues 406 to 414 (*) that further delineates the Mab-binding epitope. The 410-421 deletion mutant is also shown (|―|), together with the two cysteine residues flanking the deletion region (C) which are proposed to form a disulfide bond, and alanine 417, which, when changed to aspartic acid by point mutation, gives rise to a decrease in CD4 binding ability. The data presented by Lask et al. (Cell, 1987, 50, 975-985) implies that this polypeptide region is a critical contact site for receptor binding and it was consequently chosen as a potential candidate peptide for preparation of an immunogen to elicit catalytic antibodies that induce proteolysis of this region in the intact HIV gp 120 envelope. Introduction of a dipeptide isostere into the selected sequence, conjµgation to carrier protein, immunization, and screening for catalytic antibodies is carried out essentially as described in Examples 1-4 above. Assay of catalytic antibody proteases for their ability to interfere with the infection of target cells, in vitro, is carried out by the process described in Example 3.

### Example 6

### Detoxification of LPS during endotoxic shock Gram-negative bacterial infections

### Background

Despite the advent of antibiotics, approximately 200,000 patients develop nosocomial bacteremia resulting in about 80,000 fatalities (Maki, 1981). Gram-negative septicemia has a mortality of 30% and if shock is involved, 70% (Kreger et al., 1980). Many of the symptoms and effects, and the resistance to antibiotic therapy, are consistent with the premise that endotoxin or lipopolysaccharide (LPS) is the causative agent of the bacteria-induced shock. Some of the biological effects of LPS include hypotension, fever, intravascular coagulation, and death. LPS stimulates various cell types to release mediators, hormones, or other factors (in particular, tumor necrosis factor), which in turn provoke and affect other organs or targets. Therapeutic intervention usually includes antibiotics, but these treatments are often unsuccessful at halting the cascading effects of the LPS. New therapies are under development to neutralize the bacteria and LPS before they affect other targets.

### Bacterial products

Gram-negative bacteria have a characteristic membrane consisting of lipoprotein, lipopolysaccharide, mucocomplex, and cytoplasmic membrane layers. Several of the outer surface structures and proteins are a means by which to classify the organisms into serogroups or serotypes. For example, the capsule is used to serogroup the meningococci into groups A, B, C, etc. Another structural component that is antigenic and is used for serotyping (O-Antigen), is LPS. Polysaccharide moieties comprise the outer core and are responsible for the observed antigenic variation. The LPS also has an inner core of 1-glycero-D-mannoheptose and KDO (2-keto-3-deoxy manno-octulosonic acid) units which are common to gram-negative bacteria (except for a few mutant strains). Within the bacterial membrane is the lipid A core (I) that is conserved and composed of glucosamine disaccharide, fatty acid chains, and phosphate units. It is the lipid A core which is important structurally as well in creating the determined symptoms described previously.

Lipid X, a monosaccharide precursor to lipid A (II) is not toxic at doses greater than 3 mg for mice and 1 mg/kg in sheep, and protects mice from a lethal dose of endotoxin (Munford and Hall, 1986). This example outlines a method whereby this acyloxyacyl hydrolysis will be catalyzed by a catalytic antibody.

### Synthesis of the antigen

Scheme 1 outlines the possible synthetic reactions needed to synthesize the antigen (III), which partly follows the method used by Kusumoto et al. (1984).

### Hybridoma production and screening

The lipid X analog (III) is incorporated into liposomes of different sizes and lipid composition, or coated onto sheep erythrocytes for optimal antigen presentation before injection into BALB/c mice according to the method described by Brade et al. (1987).

Hybridomas producing antibody against the acyloxyacyl hydrolase transition state analog (III) are produced by standard methods. The hybridoma supernatant fluids are assayed for antibody which binds to the lipid X analog by ELISA and/or passive hemolysis inhibition. The catalytic activity is assayed by the methods described later.

### Assay for esterase activity

Biosynthetically labeled LPS is prepared by culturing S. typhimurium PR 122 (gal Em nag-) with [³H]-acetate and N-acetyl-1-[¹⁴C]-glucosamine (New England Nuclear Corp., Boston, Mass.). In order to generate the low molecular weight LPS (rough LPS [R-LPS]), the S. typhimurium is cultured in the absence of D-galactose. These cells are divided into aliquots, of which one is stored as a suspension in 50% glycerol at -70°C and are extracted, using phenol/chloroform/pet ether 2:5:8 (Vol/Vol). For the generation of high molecular weight LPS (smooth LPS [S-LPS]), the S. typhimurium are cultured as described above with the addition of D-galactose. These cells are divided into 2 aliquots. One is stored as whole cells in 50% glycerol at -70°C. S-LPS is extracted from the second, using 45% (wt/vol) phenol followed by dialysis against water. The two preparations of LPS are further purified by extraction with diethyl ether, and then suspended in water and triethylamine (1 µg/ml), aliquoted, and stored at -70°C.

### Biological activities of abzyme-treated LPS: in vivo tests; pyrogen assay

Treated and contol LPS are tested for pyrogenicity in rabbits. At least 3 rabbits (1.7 to 2.3 kg) are injected with the LPS preparations and their temperature changes recorded. Initially, 5 µg of the control LPS, and 50, 100, and 200 µg of the treated LPS are tested.

### Lethality test

C57BL/6 mice are injected (I.P.) with 16 mg of D-galactosamine to induce susceptibility to LPS. The treated and untreated LPS are injected (I.V.) and the LD₅₀ determined. Concentrations between 2 ng and 20 µg are tested.

### In vitro assays: macrophage spreading

Macrophages are cultured in the presence and in the absence of treated and control LPS. The percentage of macrophages spreading [which equals the (number spreading/number of total attached cells) x 100] is calculated. Macrophages are defined as spreading if their membrane apron covers an area double that of unspread cells.

### Splenocyte stimulation

Stimulation of murine splenocytes is monitored by thymidine uptake. Splenocytes of BALB/c nu/nu, C3H/HeN, and C3H/HeJ mice are incubated with [3H]-thymidine and treated and control LPS, at several concentrations. A control culture without LPS is also prepared. The stimulatory effect is expressed as a ratio of the test culture cpm to the control culture.

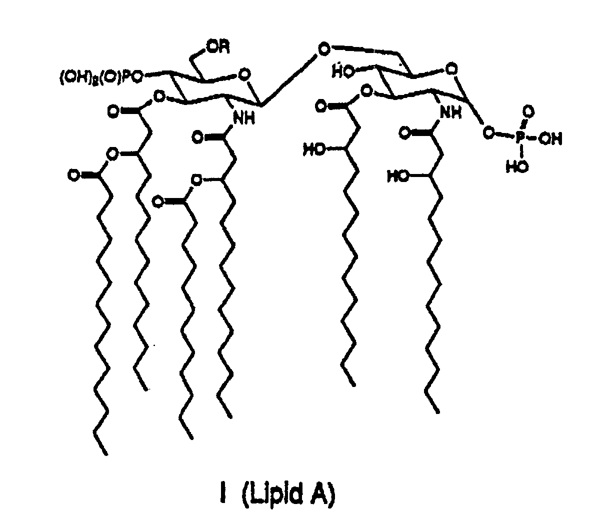

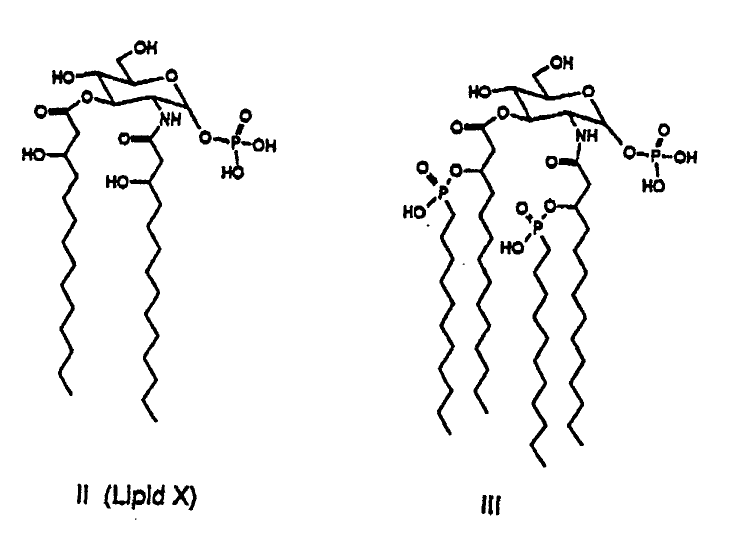

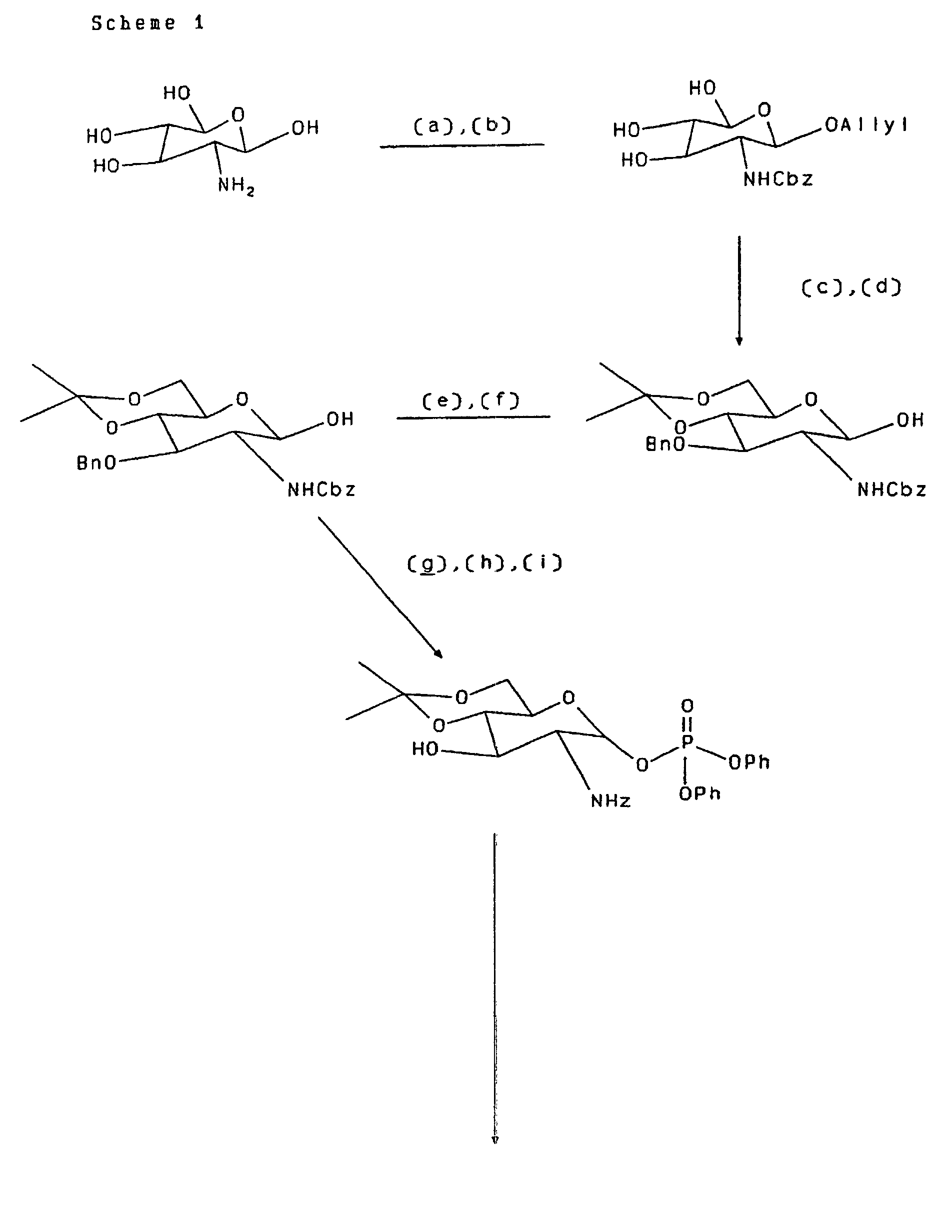

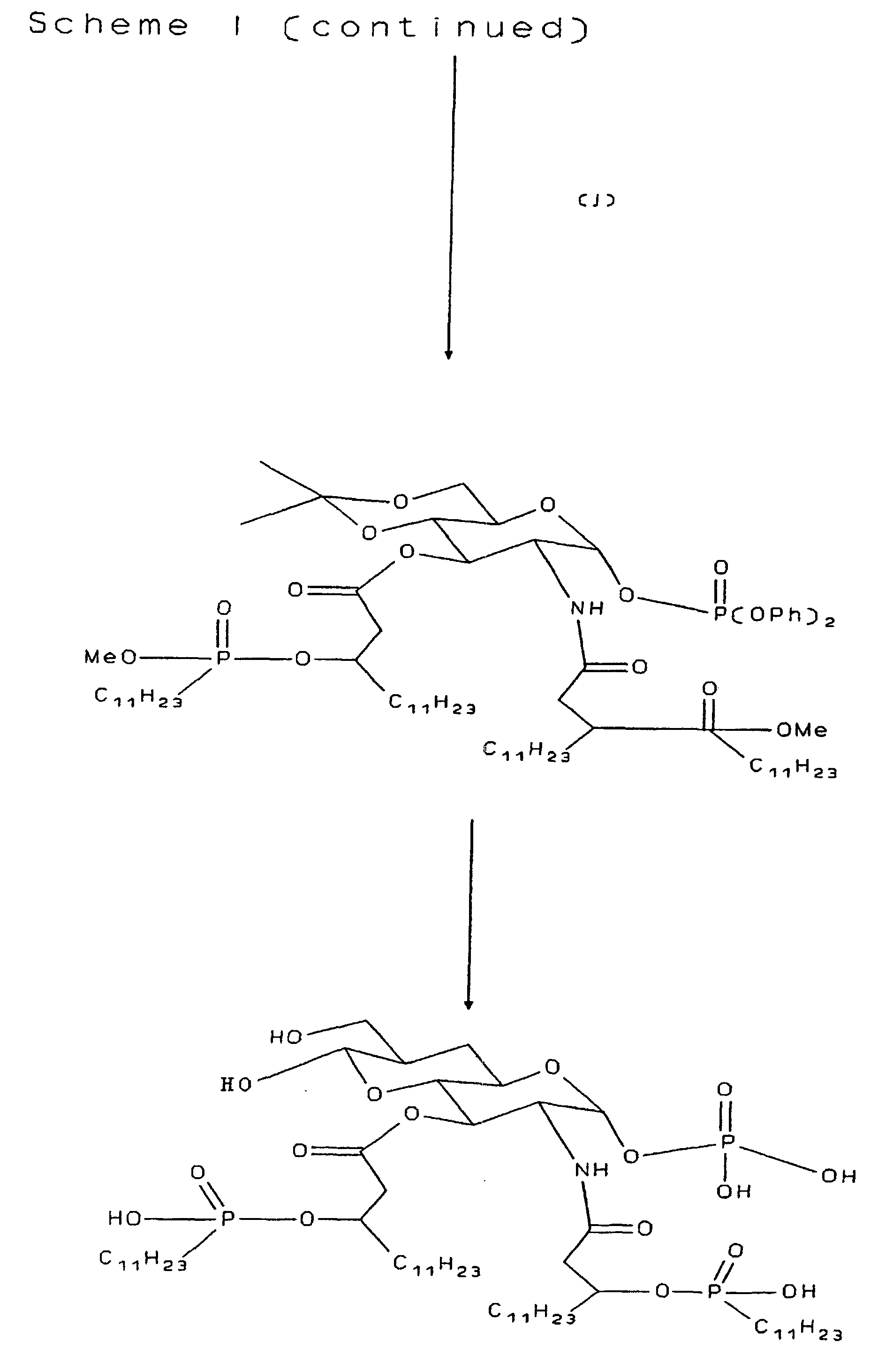

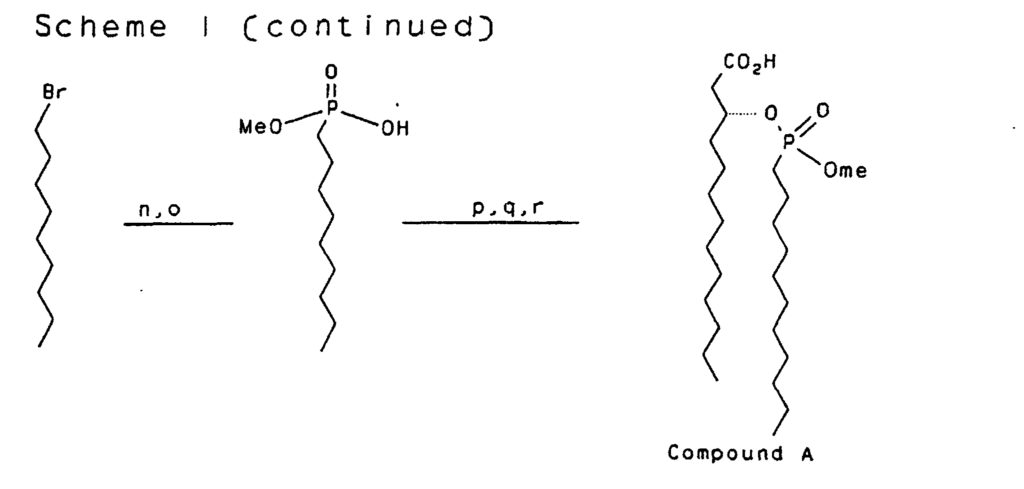

### Reagents:

(a) benzyl chloroformate/NaOH;(b) allyl atcohol/HCl; (c) acetone/TsOH/CaSO₄; (d) NaH/SnBr; (e) [Ir(COD)(PMePh₂)₂]PF₆;(f) I₂/H₂O/THF; (g) n-BuLi/-70°; (h) (PhO)₂P(O)Cl; (i) H₂/Pd/C; (j) DCCl/DMAP/Compound A ; (k) PhSH/Et₃N; (I) H₂/Pd/C/PtO₂/AcOH ; (m) HCI/THF ; (n) P(OMe)₃; (o) NaOH; (p) SOCl₂; (q) (R)-3-hydroxytetradecanoic acid; (r) H₂/Pd/C

### Example 7 Methodology for the production, screening, and isolation of monoclonal antibodies that cleave human IgE and prevent allergic reactions

### Background

The role of IgE in initiation of allergic responses has been the subject of extensive study for over 60 years. These studies have led to detailed understanding of the pathway for the allergic reaction. The primary event in the initiation of the allergic reaction is the binding of the allergen (antigen) to IgE. This results in crosslinking of the IgE on the surface of mast cells and basophil cells, the IgE being bound via its Fc region to Fc receptors present on the target cells. The consequence of this crosslinking is to trigger the release of histamine, SRS-A and other vasoactive amines which ultimately lead to the deleterious effects of an allergic response via their effect on other tissues in the body.

The IgE molecule has been functionally divided into two parts defined by their binding activities. This is demonstrated when the molecule is subjected to papain proteolysis which generates two fragments. The consequences of this proteolysis is to inactivate the IgE with respect to its ability to elicit allergic reactions. In fact, it becomes an inhibitor via blockade of Fc receptors with Fc fragments.

In order to generate an antiallergic monoclonal antibody, catalytic antibodies are prepared which are able to specifically cleave IgE and inactivate it without other deleterious effects. To achieve this, monoclonal antibodies are elicited with a cyclic dipeptide analog (Fig. 1) contained within the sequence of interest. Catalytic monoclonal antibodies so elicited will cause cleavage of the native IgE peptide sequence at the position shown: 
CRDSN¦PRGVSAYLSRPSPFDLFIRKSPTITC
¦
cleavage site
 This sequence is located between the CH2 and CH3 domains of IgE, cleavage of which will disrupt the activity of the IgE molecule and inhibit generation of an allergic response.

In order to raise a catalytic antibody to this region, the peptide hapten ADS(X)RGV, where (X) represents the cyclic dipeptide analog shown below, is synthesized by standard solid or solution phase methods. The completed peptide is fully deprotected and, if solid phase is used, cleaved from the solid support using trifluoroacetic acid. The N-terminal amino group of the peptide allows it to be attached to a carrier protein for immunization of mice.

### A. Preparation of the Immunogen

### 1. Peptide Synthesis

The peptide hapten is synthesized by the solid phase technique using the polyamide-Kieselguhr composite resin. The side chain protection groups are the following: O-tert-butyl (tyrosine, aspartic acid, glutamic acid, serine, threonine); N-4-methoxy-2,3,6,-trimethyl benzenesulphonyl (arginine). The temporary protection of the N- function is by fluorenylmethoxycarbonyl which is removed in 10 minutes with piperidine/DMF: 20/80. The coupling reactions are carried out using FMOC-amino acid anhydrides. The protected peptidyl-resin is fully deprotected by treatment with trifluoroacetic acid/thioanisole/m-cresol/thiophenol/ethanedithiol solution: 90/2/2/2/4 for three hrs. After filtration, the filtrate is concentrated under vacuum to a small volume. Ether is added to give a precipitate of the peptide. The ethereal supernatant is removed and the peptidic precipitate is washed twice with ether to yield the peptide hapten Ala-Asp-Ser-(X)-Arg-Gly-Val wherein the bracketed moiety is the cyclic dipeptide analog shown below.

### 2. Conjugation of the Hapten to the Carrier Molecule

The peptide hapten as above is conjugated to keyhole limpet hemocyanin (KLH) using glutaraldehyde. Coupling efficiency is 50-80% as estimated by binding of a trace amount of Iodine-125 labeled peptide added to the reaction mixture.

### B. Preparation and Screening of Monoclonal Antibodies

KLH conjugate peptide (50 µg) in complete Freund's adjuvant is injected into BALB/c mice. Hybridoma fusions are carried out by standard methods using SP2/0 myeloma as the fusion partner. Polyclonal antiserum response and hybridoma screening cells resulting from the fusion are screened for binding activity by ELISA.

Wells of plastic microtiter plates (Falcon 3915 Probind, Becton Dickinson Labware CAS, USA) are coated with 50 µL of peptide 5 µg/mi) in Tris-HCl buffer (0.1 M, pH 9.6).

Plates are first incubated for 30 minutes at 37°C and then overnight at room temperature. After washing three times with Tween-containing phosphate-buffered saline (PBS-Tween 0.1% pH 7.4), 50 µl of serial dilutions of antisera in PBS-BSA 1% pH 7.4 are added in peptide-coated duplicate wells and incubated for 2 hrs. at 37°C. Plates are washed three times again with PBS-Tween 0.1% and wells are then treated with 50µl of alkaline phosphatase-labeled goat anti-mouse IgG diluted 1:500 (Sigma, MO, USA). Incubation is carried out for 1 hour at 37°C.

Additional extensive washing with PBS-Tween 0.1% is followed by incubation with 150 µL of alkaline phosphatase substrate (2 tablets/10 mi of Sigma 104-105) dissolved in 0.1 M glycine-NaOH buffer (pH 10.4) containing MgCl₂ and ZnCl₂, 1 mM. The enzymatic reaction is allowed to proceed for 2 hrs. at 37°C and stopped by addition of 50 µL of Na₂CO₃ (1.5 M).

Absorbance is read at 405 nm in a Titertech Multiskan ELISA Reader (Flow Laboratories). Titer expression is determined by multiplying the optical density by the maximal dilution giving an absorbance three times as high as the negative control (consisting of pooled normal mouse sera diluted at 1:100).

Hybridomas giving a positive reaction in this screening assay are chosen for further study. IgG is purified from ascites fluid by HPLC with a Bakerbond ABₓ HPLC column.

### C. Catalysis of Peptide Cleavage by Catalytic Antibody Specific for the CH₂CH₃ Region of Human IgE

The peptide Substrate ADSNPRGV (2.7 µM) is incubated with the catalytic antibodies produced by the procedure outlined above and the reaction monitored by reverse phase HPLC analysis of the mixture. Antibodies that show catalytic peptidase activity against the peptide substrate are treated for their ability to cleave IgE.

### Assay for IgE Inactivation

Purified IgE anti-NP is subjected to digestion by the purified catalytic antibody, using 1µg of IgE with 1 µg of catalytic antibody. The incubation is carried out in PBS(pH &.5) for varying periods (hours to days) at 37°C. To control for nonspecificity in this reaction, noncatalytic monoclonal antibodies are included in parallel reactions.

To evaluate the cleavage, loss of basophil binding is studied. Samples (1-5 ng) of IgE anti-NP from the above digestion are incubated with a range of basophil cells (6x10⁵·10⁷ cells/ml) in 200 µl of RPMI 1640, 10% fetal calf serum and 10mM EDTA for 15 at 37°C. These cells are then washed 3 times in the same buffer followed by addition of ³⁵S-BSA-NIP (0.1 µCi) and further incubation for 15' at 37°C. The cells are washed and counted for radioactivity. Reduction or loss of ³⁵S binding to the cells, relative to the IgE control incubations, demonstrates that cleavage of IgE has occurred.

### CYCLIC PEPTIC ANALOG

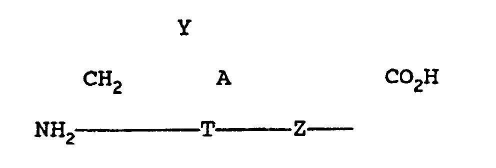

Where:
Y = 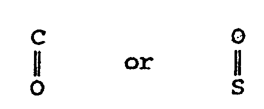
A = CH₂ or S
T = 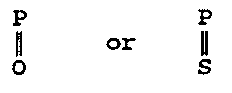
Z = N or CH

### Example 8

### Activation of a Prodrug Using a Catalytic Antibody as a Glycosidase

### Background

Antimetabolites are compounds that interfere in either the biosynthesis, utilization, or metabolic function of normal cellular metabolites. To be successfully selective in the chemotherapy of tumors, an antimetabolite should adversely affect one or more vital metabolic reactions in the tumor without seriously endangering normal tissues.

Some of the most successful anticancer drugs have been those based on purine or pyrimidine analogs whose activity is dependent on their ability to inhibit DNA or RNA synthesis. One such drug is arabinosyl cytosine (I) (cytaribine, Ara C or CA) whose activity as an inhibitor of DNA synthesis derives from the presence of arabinose in place of ribose, the difference being in the stereochemistry of the 2' hydroxy group. Ara C is administered in the free 5'-hydroxl form and only becomes activated after entry into cells by phosphorylation to the 5'-triphosphate form. Thus, it is already a prodrug, but when administered systemically, its activation can take place in any cell, tumor or normal, into which the drug enters. As a result of the wide systemic distribution of the drug, numerous side effects occur, such as nausea, vomiting, alopecia, myelosuppression, etc.

It has now been found that Ara C can be modified to a prodrug form in which spontaneous intracellular activation would be reduced. First, an antitumor monoclonal antibody is targeted to the tumor, carrying with it a catalytic antibody, the two being either chemically or genetically linked. Second, the pro form of Ara C is administered and its activation is then restricted to those tissues bearing the abzyme activity. Thus a more favorable discrimination of tumor and normal tissue results. Since Ara C has a very short plasma half-life, diffusion of the activated drug away from the tumor site is followed by rapid deactivation before significant systemic toxicity results.

### Synthesis of 5'-galactosyl Ara C and its transition state analog

The synthesis of the amidine galactosyl analog of Ara C (15) is outlined in Schemes 2 and 3 below. The tribenzoylated derivative (3) from Scheme 1 is converted to (9) by treatment with methanesulfonyl chloride in pyridine followed by displacement with lithium azide in the N,NM-dimethylformamide at 75°C. Hydrogenation of (9) in ethanol at 50 psi of hydrogen pressure in the presence of 10% palladium on charcoal affords the 4'amino derivative.

The β-galactonolactam (13) is prepared by first treating 2,3,4,6-tetra-D-benzyl-2-d-galactopyranose (11) with dimethyl sulfoxide and acetic anhydride to give 2,3,4,6,-tetra-O-benzyl-D-Galactono-1,5-lactone (12) which is then condensed with aqueous ammonia (25% w/w) solution in the presence of trace amounts of Amberlite 1R 120 H⁺ in dioxane for 6 hrs to afford (13). Conversion of (13) to its imido ester analog by treating it with trimethyloxonium tetrafluorobarate followed by its reaction the 5'amino derivative (10) yields the fully protected amidine galactosyl analog (14). Deprotection using hydrogenation at 50 psi of hydrogen pressure in the presence of 10% palladium on charcoal followed by treatment with concentrated aqueous ammonia gives (15).

The synthesis of the 5'-beta-D-galactose analog of cytosine-beta-D-Arabinofuranoside (5) is outlined in Scheme 1.

Treatment of Ara C (1) with Bis (P-methoxyphenyl) phenyl methyl chloride in pyridine, followed by tribenzoylation using benzoyl chloride and then detritylation of (2) with trichloroacetic acid in dichloromethane, affords the partially protected derivative (3). Dilute aqueous acid treatment of beta-D-galactose pentaacetate (6) followed by treatment with sodium hydride and excess trichloroacetonitrile yields the trichloroacetimidate (8). Coupling of (8) with (3) in the presence of the Lewis acid boron trifluoride etherate in dichloromethase gives (4). The 5'-beta-D-galactose analog of Ara C (5) is obtained after the complete deprotection of (4) using concentrated aqueous ammonia.

### Production of antibodies and screening for Binding to Transition State Analogs

Monoclonal antibodies to (15) in Scheme 4, after conjugation to a suitable carrier, are produced essentially as described in Example 1. Antibody-producing clones are first screened for their ability to bind to the Ara C analog (15) by methods similar to those described in Example 1.

### In vitro catalytic activity assay

Those antibody clones displaying binding activity for (15) are screened for their ability to cleave the galactosyl moiety from the Ara C substrate (5) by an assay essentially as described in Koerner and Nieman (J. Chromatography 449, 216-228 (1988), but substituting galactose oxidase for glucose oxidase. The principle of the assay is the detection of galactose as it is released from the prodrug by the catalytic antibody using a galactose oxidase/luminol chemiluminescence procedure.

### In vivo assays

The conversion of galactosyl Ara C to Ara C by the catalytic antibody in the presence of target cells results in inhibition of DNA synthesis and cell-killing. A simple assay of DNA synthesis is carried out essentially as described in Gish et al. (J. Med. Chem 14, 1159-1162, 1971), in which the ablity of Ara C to inhibit DNA synthesis in phytohaemagglutin (PHA) stimulated human lymphocytes, using a tritiated thymidine incorporation assay, is measured.

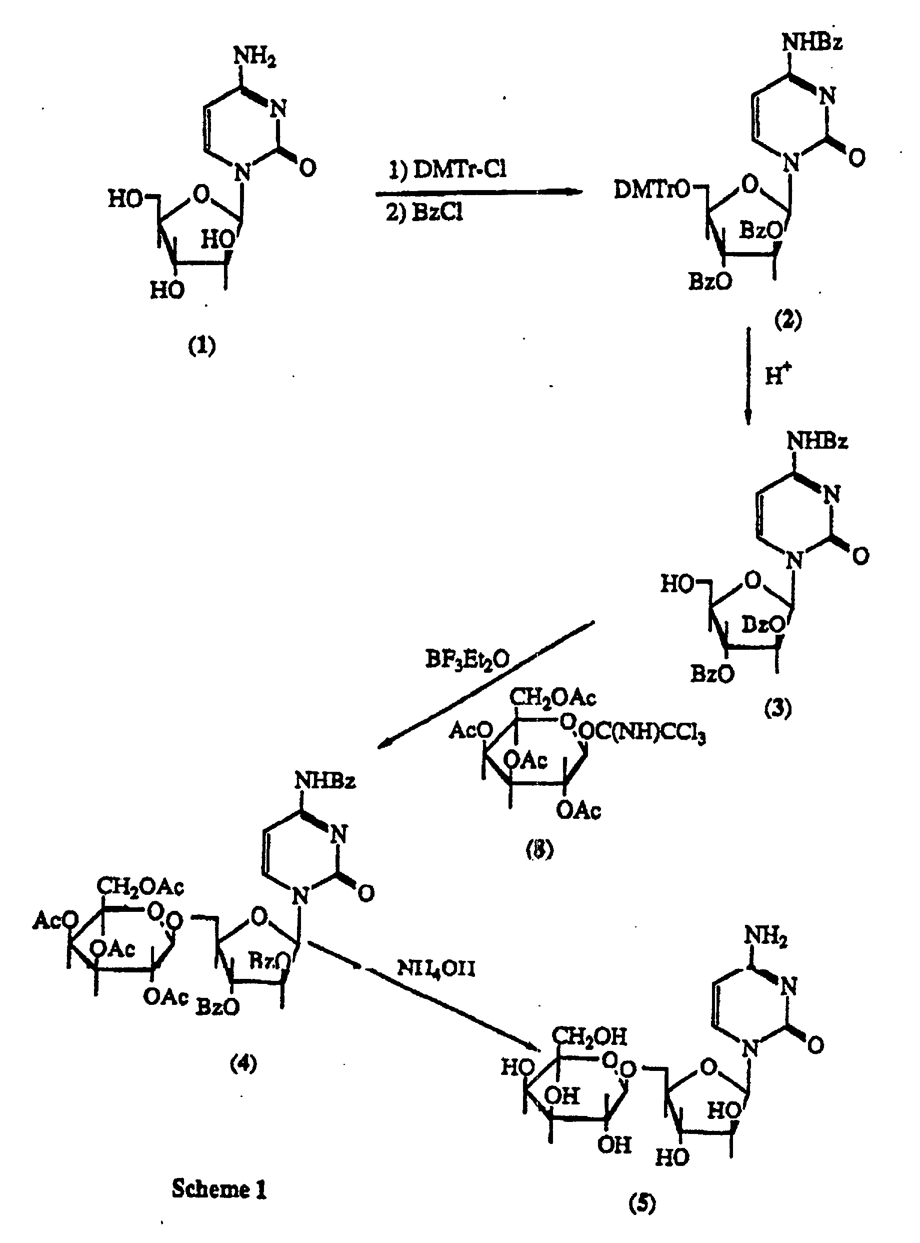

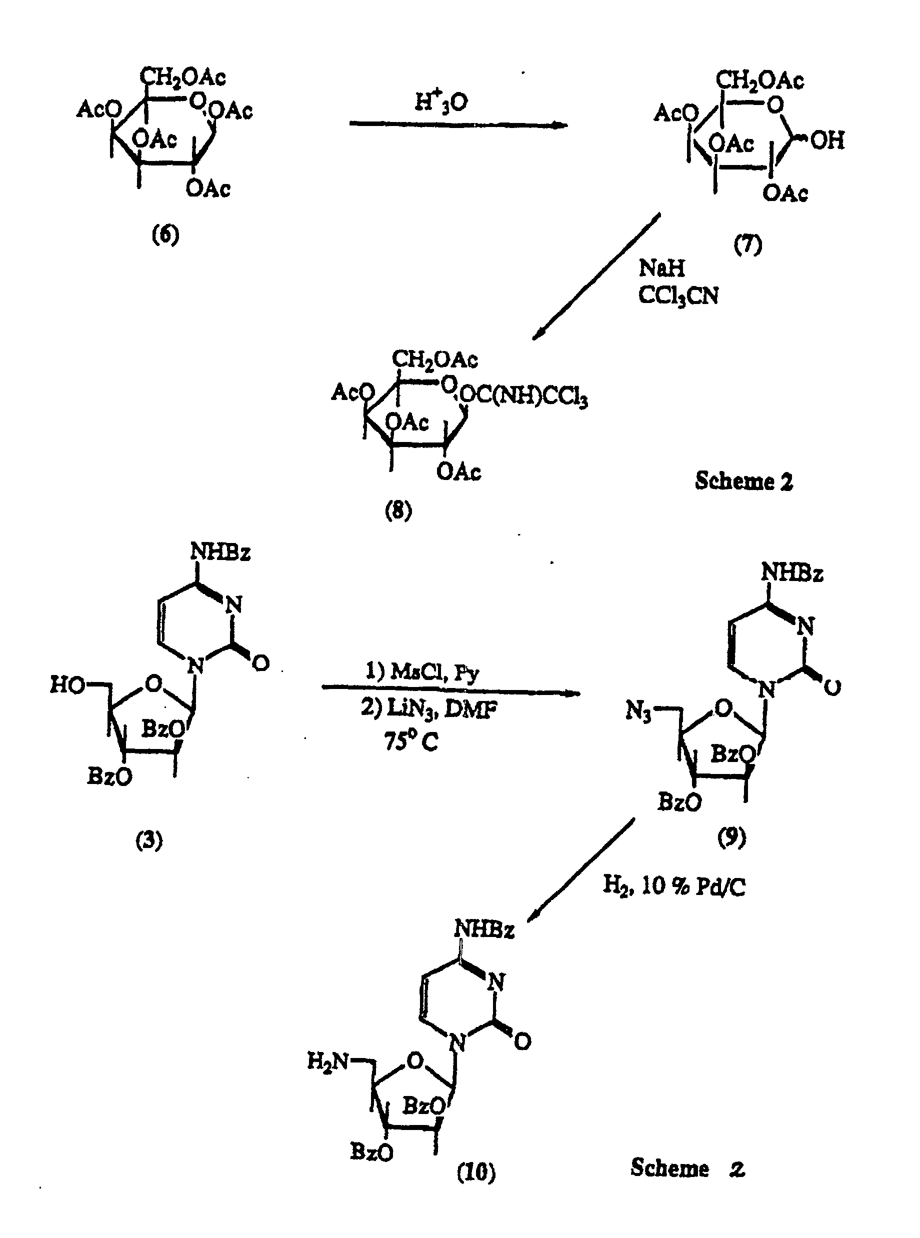

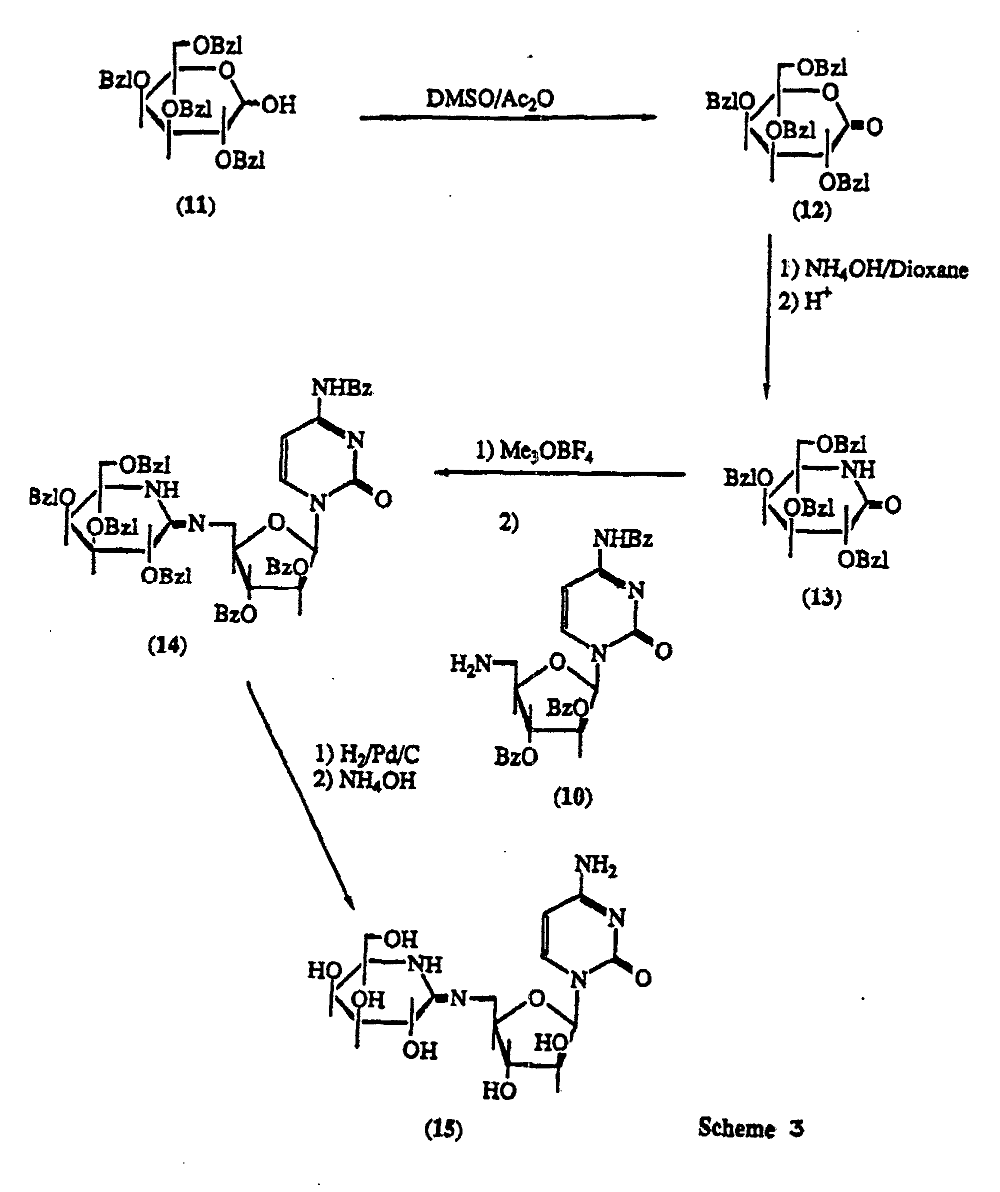

### REFERENCES

1. The Chemistry of Enzyme Action, Chapter 1, M.I. Page, Editor (Elsevier, Amsterdam 1984).
2. A.D. Napper et al., "A Stereospecific Cyclization Catalyzed by an Antibody," Science, 237, 1041-1043 (1987).
3. A. Tramontano et al., "Chemical Reactivity at an Antibody Binding Site Elicited By Mechanistic Design of a Synthetic Antigen," Proc. Nat'l Acad. Sci. USA, 83, 6736-6740 (1986).
4. H. White and W.P. Jencks, J. Biol. Chem., 252, p. 1688 (1976); H. White et al., ibid, 1700.
5. H.M. Geysen et al., J. Immunoloaical Methods, 102, 259-274 (1987).
6. H.M. Geysen et al., Proc. Nat'l Acad. Sci. USA, 82, 178-182 (1985).
7. J.A. Berzofsky, Science, 229, 932-940 (1985).
9. T.P. Hopp and K.R. Woods, Proc. Nat'l. Acad. Sci, USA, 78, 3824-3828 (1981).
9. J. Novotny et al., Proc. Nat'l Acad. Sci., 226 (1986).
10. H.M. Geysen et al., Science, 235, 1184 (1878).
11. A. Fersht, Enzyme Structure and Mechanism, 2d ed., 433-436 (W.H. Freeman and Co., New York, 1985).
12. J. Blundell et al., Nature, 304, 273-275 (1983).
13. P. Corvol et al., J. Biol. Chem., 262(6), 2913-2918 (1987).
14. E. Atherton and R.J. Sheppard, J. Chem. Soc. Commun., 1151-1152 (1981).
15. M. Wilchek and S. Banniger, Methods Enzymol., 70, 151-159 (1980).
16. J. Menard and K.J. Catt, Endocrinology, 90, 422-430 (1972).
17. C.B. Pert et al., Proc. Nat'l. Acad. Sci, USA, 83, 9254-9258 (1986).
18. M.R. Pincus et al., Biochem. Biophys. Res. Commun., 143(a), 248-251 (1987).
19. D.M. Katz et al., Biochemistry, 18, 690-697 (1979).
20. Anderson et al., CRU, 10 27 (1977).
21. M.D. Schaff et al., Cell, 8, 405 (1976).
22. C. Milstein et al., Nature, 266, 550 (1977).
23. J.W. Littlefield, Expt'l. Cell Res., 41, 190 (1966).
24. D. Ho, J. Virol., 61, 2024 (1987).
25. B.D. Walker et al., Proc. Nat'l Acad. Sci. USA, 84, 8120 (1987).

## Claims

1. The use of an effective amount of a rate-enhancing antibody in the manufacture of a pharmaceutical composition for the treatment of diseases treatable by enhancing the rate of cleavage or formation of a specific amide, peptide, ester or glycosidic bond within a biomolecule *in vivo*, said antibody having been produced by the method of:
a) selecting the specific amide, peptide, ester or glycosidic bond to be cleaved or formed;
b) selecting an antigen comprising an analog of said biomolecule and containing an analog of said bond to be cleaved or formed;
c) exposing cells capable of producing antibodies to said antigen and thereby generating antibody producing cells;
d) hybridizing said antibody producing cells with myeloma cells and thereby generating a plurality of hybridoma cells each producing monoclonal antibodies; and
e) screening said plurality of monoclonal antibodies to identify a monoclonal antibody which enhances the cleavage or formation of said amide, peptide, ester or glycosidic bond.

2. The use in the manufacture of a pharmaceutical for the treatment of diseases treatable by enhancing the rate of cleavage or formation of a specific amide, peptide, ester or glycosidic bond within a biomolecule *in vivo*, of an effective amount of an antigen capable of eliciting antibodies in an animal which enhance the rate of cleavage or formation of said bond, said antibody having been produced by the method of:
a) selecting the specific amide, peptide, ester or glycosidic bond to be cleaved or formed;
b) selecting an antigen comprising an analog of said biomolecule and containing an analog of said bond to be cleaved or formed;
c) exposing cells capable of producing antibodies to said antigen and thereby generating antibody producing cells;
d) hybridizing said antibody producing cells with myeloma cells and thereby generating a plurality of hybridoma cells each producing monoclonal antibodies; and
e) screening said plurality of monoclonal antibodies to identify a monoclonal antibody which enhances the cleavage or formation of said amide, peptide, ester or glycosidic bond.

3. A use as recited in Claim 2 wherein said antigen comprises an analog of said biomolecule containing an analog of said bond to be cleaved or formed.

4. The use of an effective amount of a rate-enhancing antibody in the manufacture of a pharmaceutical composition for the treatment of diseases treatable by activating or deactivating a biological function in an animal by enhancing the rate of cleavage or formation of a specific amide, peptide, ester or glycosidic bond within a biomolecule *in vivo*, said antibody having been produced by the method of:
a) identifying a biomolecule whose formation or cleavage leads to the activation or deactivation of the desired biological function, said biomolecule containing an accessible bond to be cleaved or formed;
b) selecting an antigen which comprises an analog of the said biomolecule containing an analog of said bond to be cleaved or formed;
c) exposing cells capable of producing antibodies to said antigen and thereby generating antibody producing cells;
d) hybridizing said antibody producing cells with myeloma cells and thereby generating a plurality of hybridoma cells each producing monoclonal antibodies; and
e) screening said plurality of monoclonal antibodies to identify a monoclonal antibody which enhances the cleavage or formation of said amide, peptide, ester or glycosidic bond.

5. A use as recited in Claim 4 wherein the bond to be cleaved or formed is determined to be accessible or surface-located by reference to the 3-dimensional structure of said biomolecule.

6. A use as recited in Claim 4 wherein the bond to be cleaved or formed is determined to be accessible or surface-located by computer modelling or predicted structures.

7. A use as recited in Claim 4 wherein the bond to be cleaved or formed is determined to be accessible or surface-located by analogy to homologous compounds.

8. A use as recited in Claim 4 wherein the bond to be cleaved or formed is determined to be accessible or surface-located by location of a high proportion of hydrophillic or charged amino acids.

9. The use in. the manufacture of a pharmaceutical for the treatment of diseases treatable by activating or deactivating a biological function in an animal by enhancing the rate of cleavage or formation of a specific amide, peptide, ester or glycosidic bond within a biomotecule *in vivo*, of an effective amount of an antigen capable of eliciting antibodies in said animal which enhances the rate of cleavage or formation of said bond, said antigen having been produced by the method of:
a) identifying a biomolecule whose formation or cleavage leads to the activation or deactivation of the desired biological function, said biomolecule containing an accessible or surface-located bond to be cleaved or formed; and
b) selecting an antigen which comprises an analog of the said biomolecule containing an analog of said bond to be cleaved or formed.

10. A use as recited in Claim 9 wherein the bond to be cleaved or formed is determined to be accessible or surface-located by reference to the 3-dimensional structure of said sequence.

11. A use as recited in Claim 9 wherein the bond to be cleaved or formed is determined to be accessible or surface-located by computer modelling or predicted structures.

12. A use as recited in Claim 9 wherein the bond to be cleaved or formed is determined to be accessible or surface-located by analogy to homologous proteins.

13. A use as recited in Claim 9 wherein the bond to be cleaved or formed is determined to be accessible or surface-located by location of a high proportion of hydrophillic or charged amino acids.

14. A use as recited in Claim 9 wherein a peptide or protein to be cleaved or formed includes an amino acid sequence selected from the group consisting of ASN-GLY, ASN-PRO, ASP-GLY, ASP-PRO, GLN-X and GLU-X, where X is any amino acid.

15. The use according to any preceding Claim wherein said antibody is adapted to cleave a bond in a molecule selected from renin, a viral coat protein, LPS, TNF and IgE.

16. The use according to Claim 15, wherein the viral coat protein is gp120.

## Patentansprüche

1. Die Verwendung einer wirksamen Menge eines die Geschwindigkeit erhöhenden Antikörpers für die Herstellung einer pharmazeutischen Zusammensetzung für die Behandlung von Krankheiten, die behandelbar sind durch Erhöhen der Geschwindigkeit der Spaltung oder Bildung einer spezifischen Amid-, Peptid-, Esteroder Glykosidbindung in einem Biomolekül *in vivo*, wobei dieser Antikörper hergestellt wurde durch das Verfahren der:
a) Auswahl der zu spaltenden oder zu bildenden spezifischen Amid-, Peptid-, Ester- oder Glykosidbindung;
b) Auswahl eines Antigens, das ein Analogen dieses Biomoleküls enthält und das ein Analogon dieser zu bildenden oder zu spaltenden Bindung enthält;
c) Aussetzen von Zellen, die fähig sind, Antikörper zu produzieren, an dieses Antigen und dadurch Erzeugen von antikörperproduzierenden Zellen;
d) Hybridisieren dieser antikörperproduzierenden Zellen mit Myelomzellen und dadurch Erzeugen einer Vielzahl an Hybridomzellen, von denen jede monoklonale Antikörper erzeugt; und
e) Screenen dieser Vielzahl an monoklonalen Antikörpern, um einen monoklonalen Antikörper zu identifizieren, der die Spaltung oder Bildung dieser Amid-, Peptid-, Ester- oder Glykosidbindung verstärkt.

2. Die Verwendung für die Herstellung eines Arzneimittels für die Behandlung von Krankheiten, die behandelbar sind durch Erhöhen der Geschwindigkeit der Spaltung oder Bildung einer spezifischen Amid-, Peptid-, Ester- oder Glykosidbindung in einem Biomolekül *in vivo*, von einer wirksamen Menge eines Antigens, das fähig ist Antikörper in einem Tier hervorzurufen, die die Geschwindigkeit der Spaltung oder Bildung dieser Bindung erhöhen, wobei der Antikörper hergestellt wurde durch das Verfahren der:
a) Auswahl der zu spaltenden oder zu bildenden spezifischen Amid-, Peptid-, Ester- oder Glykosidbindung;
b) Auswahl eines Antigens, das ein Analogon dieses Biomoleküls enthält und das ein Analogon dieser zu bildenden oder zu spaltenden Bindung enthält;
c) Aussetzen von Zellen, die fähig sind, Antikörper zu produzieren, an dieses Antigen und dadurch Erzeugen von antikörperproduzierenden Zellen;
d) Hybridisieren dieser antikörperproduzierenden Zellen mit Myelomzellen und dadurch Erzeugen einer Vielzahl an Hybridomzellen, von denen jede monoklonale Antikörper erzeugt; und
e) Screenen dieser Vielzahl an monoklonalen Antikörpern, um einen monoklonalen Antikörper zu identifizieren, der die Spaltung oder Bildung dieser Amid-, Peptid-, Ester- oder Glykosidbindung verstärkt.

3. Eine Verwendung wie in Anspruch 2, in welcher dieses Antigen ein Analogon dieses Biomoleküls enthält, das ein Analogon dieser zu spaltenden oder zu bildenden Bindung enthält.

4. Die Verwendung einer wirksamen Menge eines die Geschwindigkeit erhöhenden Antikörpers für die Herstellung einer pharmazeutischen Zusammensetzung für die Behandlung von Krankheiten, die behandelbar sind durch Aktivieren oder Deaktivieren einer biologischen Funktion in einem Tier durch Erhöhen der Geschwindigkeit der Spaltung oder Bildung einer spezifischen Amid-, Peptid-, Ester- oder Glykosidbindung in einem Biomolekül *in vivo*, wobei dieser Antikörper hergestellt wurde durch das Verfahren der
a) Identifizierung eines Biomoleküls, dessen Bildung oder Spaltung zu der Aktivierung oder Deaktivierung der gewünschten biologischen Funktion führt, wobei dieses Biomolekül eine zugängliche zu spaltende oder zu bildende Bindung aufweist;
b) Auswahl eines Antigens, das ein Analogon dieses Biomoleküls enthält, das ein Analogon dieser zu bildenden oder zu spaltenden Bindung enthält;
c) Aussetzen von Zellen, die fähig sind, Antikörper zu produzieren, an dieses Antigen und dabei Erzeugen von antikörperproduzierenden Zellen;
d) Hybridisieren dieser antikörperproduzierenden Zellen mit Myelomzellen und dadurch Erzeugen einer Vielzahl an Hybridomzellen, von denen jede monoklonale Antikörper erzeugt; und
e) Screenen dieser Vielzahl an monoklonalen Antikörpern, um einen monoklonalen Antikörper zu identifizieren, der die Spaltung oder Bildung dieser Amid-, Peptid-, Ester- oder Glykosidbindung verstärkt.

5. Eine Verwendung wie in Anspruch 4, in welcher die zu spaltende oder zu bildende Bindung als zugänglich oder oberflächenlokalisiert ermittelt wird unter Bezugnahme auf die dreidimensionale Struktur dieses Biomoleküls.

6. Eine Verwendung wie in Anspruch 4, in welcher die zu spaltende oder zu bildende Bindung als zugänglich oder oberflächenlokalisiert ermittelt wird durch rechnergestütztes Modellieren oder über vorhergesagte Strukturen.

7. Eine Verwendung wie in Anspruch 4, in welcher die zu spaltende oder zu bildende als zugänglich oder oberflächenlokalisiert ennittelt wird durch Analogie zu homologen Verbindungen.

8. Eine Verwendung wie in Anspruch 4, in welcher die zu spaltende oder zu bildende Bindung als zugänglich oder oberflächenlokalisiert ermittelt wird durch Lokalisieren eines großen Anteils an hydrophilen oder geladenen Aminosäuren.

9. Die Verwendung für die Herstellung eines Arzneimittels für die Behandlung von Krankheiten, die behandelbar sind durch Aktivieren oder Deaktivieren einer biologischen Funktion durch Erhöhen der Geschwindigkeit der Spaltung oder Bildung einer spezifischen Amid-, Peptid-, Ester- oder Glykosidbindung in einem Biomolekül *in vivo,* von einer wirksamen Menge eines Antigens, das fähig ist, Antikörper in diesem Tier hervorzurufen, die die Geschwindigkeit der Spaltung oder Bildung dieser Bindung erhöhen, wobei dieses Antigen hergestellt wurde durch das Verfahren der:
a) Identifizierung eines Biomoleküls, dessen Bildung oder Spaltung zu der Aktivierung oder Deaktivierung der gewünschten biologischen Funktion führt, wobei dieses Biomolekül eine zugängliche oder oberflächenlokalisierte, zu spaltende oder zu bildende Bindung aufweist; und
b) Auswahl eines Antigens, das ein Analogon dieses Biomoleküls enthält, das ein Analogon dieser zu bildenden oder zu spaltenden Bindung enthält.

10. Eine Verwendung wie in Anspruch 9, in welcher die zu spaltende oder zu bildende Bindung als zugänglich oder oberflächenlokalisiert ermittelt wird unter Bezugnahme auf die dreidimensionale Struktur dieses Biomoleküls.

11. Eine Verwendung wie in Anspruch 9, in welcher die zu spaltende oder zu bildende Bindung als zugänglich oder oberflächenlokalisiert ermittelt wird durch rechnergestütztes Modellieren oder über vorhergesagte Strukturen.

12. Eine Verwendung wie in Anspruch 9, in welcher die zu spaltende oder zu bildende als zugänglich oder oberflächenlokalisiert ermittelt wird durch Analogie zu homologen Proteinen.

13. Eine Verwendung wie in Anspruch 9, in welcher die zu spaltende oder zu bildende Bindung als zugänglich oder oberflächenlokalisiert ermittelt wird durch Lokalisieren eines großen Anteils an hydrophilen oder geladenen Aminosäuren.

14. Eine Verwendung wie in Anspruch 9, in welcher ein zu spaltendes oder zu bildendes Peptid oder Protein eine Aminosäuresequenz enthält, die ausgewählt ist aus der Gruppe bestehend aus ASN-GLY, ASN-PRO, ASP-GLY, ASP-PRO, GLN-X und GLU-X, wobei X irgendeine Aminosäure ist.

15. Die Verwendung nach einem der vorstehenden Ansprüche, in welcher dieser Antikörper angepasst ist, eine Bindung in einem Molekül zu schneiden, das ausgewählt ist aus Renin, einem viralen Hüllprotein, LPS, TNF und IgE.

16. Die Verwendung nach Anspruch 15, in welcher das virale Hüllprotein gp120 ist.

## Revendications

1. Utilisation d'une quantité efficace d'un anticorps augmentant le taux, dans la fabrication d'une composition pharmaceutique destinée au traitement de maladies pouvant être traitées par une augmentation du taux de clivage ou de formation d'une liaison spécifique amide, peptide, ester ou glycosidique au sein d'une biomolécule *in vivo*, ledit anticorps ayant été produit par le procédé de :
(a) sélection de la liaison spécifique amide, peptide, ester ou glycosidique devant être clivée ou formée ;
(b) sélection d'un antigène comprenant un analogue de ladite biomolécule, et contenant un analogue de ladite liaison devant être clivée ou formée ;
(c) exposition audit antigène de cellules capables de produire des anticorps et ainsi génération de cellules produisant des anticorps ;
(d) hybridation desdites cellules produisant des anticorps avec des cellules myélomateuses et ainsi génération d'une pluralité de cellules d'hybridome produisant chacune des anticorps monoclonaux ; et
(e) criblage de ladite pluralité d'anticorps monoclonaux afin d'identifier un anticorps monoclonal qui augmente le clivage ou la formation de ladite liaison amide, peptide, ester ou glycosidique.

2. Utilisation, dans la fabrication d'un produit pharmaceutique destiné au traitement de maladies pouvant être traitées par une augmentation du taux de clivage ou de formation d'une liaison spécifique amide, peptide, ester ou glycosidique au sein d'une biomolécule *in vivo*, d'une quantité efficace d'un antigène capable, chez un animal, de déclencher la production d'anticorps qui augmentent le taux de clivage ou de formation de ladite liaison, ledit anticorps ayant été produit par le procédé de :
(a) sélection de la liaison spécifique amide, peptide, ester ou glycosidique devant être clivée ou formée ;
(b) sélection d'un antigène comprenant un analogue de ladite biomolécule, contenant un analogue de ladite liaison devant être clivée ou formée ;
(c) exposition audit antigène de cellules capables de produire des anticorps et ainsi génération de cellules produisant des anticorps ;
(d) hybridation desdites cellules produisant des anticorps avec des cellules myélomateuses et ainsi génération d'une pluralité de cellules d'hybridome produisant chacune dès anticorps monoclonaux ; et
(e) criblage de ladite pluralité d'anticorps monoclonaux afin d'identifier un anticorps monoclonal qui augmente le clivage ou la formation de ladite liaison amide, peptide, ester ou glycosidique.

3. Utilisation selon la revendication 2, dans laquelle ledit antigène comprend un analogue de ladite biomolécule, contenant un analogue de ladite liaison devant être clivée ou formée.

4. Utilisation d'une quantité efficace d'un anticorps augmentant le taux, dans la fabrication d'une composition pharmaceutique destinée au traitement de maladies pouvant être traitées par l'activation ou la désactivation d'une fonction biologique dans un animal, en augmentant le taux de clivage ou de formation d'une liaison spécifique amide, peptide, ester ou glycosidique au sein d'une biomolécule *in vivo*, ledit anticorps ayant été produit par le procédé de :
(a) identification d'une biomolécule dont la formation ou le clivage conduit à l'activation ou à la désactivation de la fonction biologique souhaitée, ladite biomolécule contenant une liaison accessible devant être clivée ou formée ;
(b) sélection d'un antigène comprenant un analogue de ladite biomolécule, contenant un analogue de ladite liaison devant être clivée ou formée ;
(c) exposition audit antigène de cellules capables de produire des anticorps et ainsi génération de cellules produisant des anticorps ;
(d) hybridation desdites cellules produisant des anticorps avec des cellules myélomateuses et ainsi génération d'une pluralité de cellules d'hybridome produisant chacune des anticorps monoclonaux ; et
(e) criblage de ladite pluralité d'anticorps monoclonaux afin d'identifier un anticorps monoclonal qui augmente le clivage ou la formation de ladite liaison amide, peptide, ester ou glycosidique.

5. Utilisation selon la revendication 4, dans laquelle la liaison devant être clivée ou formée est déterminée comme étant accessible ou située à la surface par référence à la structure tridimensionnelle de ladite biomolécule.

6. Utilisation selon la revendication 4, dans laquelle la liaison devant être clivée ou formée est déterminée comme étant accessible ou située à la surface par modélisation informatique ou structures prédites.

7. Utilisation selon la revendication 4, dans laquelle la liaison devant être clivée ou formée est déterminée comme étant accessible ou située à la surface par analogie avec des composés homologues.

8. Utilisation selon la revendication 4, dans laquelle la liaison devant être clivée ou formée est déterminée comme étant accessible ou située à la surface par localisation d'une grande proportion d'acides aminés hydrophiles ou chargés.

9. Utilisation, dans la fabrication d'un produit pharmaceutique destiné au traitement de maladies pouvant être traitées par l'activation ou la désactivation d'une fonction biologique dans un animal en augmentant le taux de clivage ou de formation d'une liaison spécifique amide, peptide, ester ou glycosidique au sein d'une biomolécule *in vivo*, d'une quantité efficace d'un antigène capable, chez ledit animal, de déclencher la production d'anticorps qui augmentent le taux de clivage ou de formation de ladite liaison, ledit antigène ayant été produit par le procédé de :
(a) identification d'une biomolécule dont la formation ou le clivage conduit à l'activation ou à la désactivation de la fonction biologique souhaitée, ladite biomolécule contenant une liaison accessible ou située à la surface devant être clivée ou formée ; et
(b) sélection d'un antigène comprenant un analogue de ladite biomolécule, contenant un analogue de ladite liaison devant être clivée ou formée.

10. Utilisation selon la revendication 9, dans laquelle la liaison devant être clivée ou formée est déterminée comme étant accessible ou située à la surface par référence à la structure tridimensionnelle de ladite séquence.

11. Utilisation selon la revendication 9, dans laquelle la liaison devant être clivée ou formée est déterminée comme étant accessible ou située à la surface par modélisation informatique ou structures prédites.

12. Utilisation selon la revendication 9, dans laquelle la liaison devant être clivée ou formée est déterminée comme étant accessible ou située à la surface par analogie avec des protéines homologues.

13. Utilisation selon la revendication 9, dans laquelle la liaison devant être clivée ou formée est déterminée comme étant accessible ou située à la surface par localisation d'une grande proportion d'acides aminés hydrophiles ou chargés.

14. Utilisation selon la revendication 9, dans laquelle un peptide ou une protéine devant être clivés ou formés incluent une séquence d'acides aminés sélectionnée dans le groupe consistant en ASN-GLY, ASN-PRO, ASP-GLY, ASP-PRO, GLN-X et GLU-X, où X est un acide aminé quelconque.

15. Utilisation selon l'une quelconque des précédentes revendications, dans laquelle ledit anticorps est adapté au clivage d'une liaison dans une molécule sélectionnée parmi la rénine, une protéine d'enveloppe virale, un LPS, un TNF et une IgE.

16. Utilisation selon la revendication 15, dans laquelle la protéine d'enveloppe virale est la gp120.
